# EUROPEAN PATENT APPLICATION

(11) **EP 1 586 642 A2**
(43) Date of publication of application: **19.10.2005**
(21) Application number: 05005471.7
(22) Date of filing: 12.10.1994
(51) Int. Cl.: C12N 15/12, G01N 33/53, C07K 16/18, A61K 38/19, A61K 39/395, C12Q 1/68, C07K 14/47, A61K 48/00

(54) **Tumor supressor genes, proteins encoded thereby and use of said genes and proteins**

(30) Priority: 12.10.1993 IL 10725093
(62) Divisional of application: 95900994.5
(71) Applicant: YEDA RESEARCH & DEVELOPMENT COMPANY, LTD., 76100 Rehovot (IL)
(72) Inventor: Kimchi, Adi, 43561 Raanana (IL)
(74) Representative: Desaix, Anne

(57) **Abstract**

The present invention is based on the pioneering finding that inhibition of expression of certain genes counteracts the cytokine-induced cell death. As long as these genes function normally, cytokine induces cell death; once the expression of said genes is inhibited, the cytokine-induced cell death is inhibited. Its follows therefrom that the normal expression is cytokine-induced cell death. In HeLa cells IFN-gamma induces a biphasic process, which comprises an initial cytostatic phase and a subsequent cytotoxic phase (programmed cell death). The novels genes discovered in accordance with the present invention were found to affect only the later, cytotoxic phase. These genes, referred to as "DAP" (death-associated protein) genes, include DNA molecules which comprise a coding sequence encoding the expression products of the DAP genes or expression products having a similar biological activity.

## Description

### FIELD OF THE INVENTION

The present invention relates to the field of tumor-suppressor genes in general, and cytokine-induced cell death in particular.

### BACKGROUND OF THE INVENTION

One of the factors which determines the proliferation state of cells is the balance between the growth-promoting effects of proto-oncogenes, and the growth-constraining effects of tumor-suppressor genes.

One mechanism by which these tumor-suppressor genes exert their growth-constraining effect is by inducing the cell to undergo a physiological type of death. Such a controlled cell death is evident in a multitude of physiological conditions including metamorphosis, synaptogenesis of neurons, death of lymphocytes during receptor repertoire selection, controlled homeostasis in the bone-marrow and other proliferative tissues, and others. Such cell death is regulated by the interaction of the cell with other cells or with cell products, for example through the activity of suitable cytokines.

Genetic mutation that inactivates the suppressor genes, liberate the cell from normal growth constraint imposed by other cells, resulting in an uncontrolled growth of the cell without any relation to external signals. This uncontrolled growth is a step in tumorigenesis.

To date, only a few tumor-suppressor genes have been fully
characterized including the *retinoblastoma (Rb)* gene, *p53, DCC, NM23 WT-1, NF-1, APC,* and *ras suppressor* genes. A mutation in either of the above genes, probably in both alleles, which leads to either blockage of expression, or production of a faulty protein, hampers the normal control of growth and viability of cells and may thus give rise to cancer.

Growth-inhibiting cytokines have a double effect on the target cell. They can either inhibit the proliferation of the cell, and/or give rise to cell death. To date, blockage or activation of expression of known tumor-suppressor genes was shown to counteract or enhance, respectively, cytokines' inhibition of cells' growth (reviewed by A. Kimchi, 1992, *J. Cell Biochem*., 50:1-9) but did not have any effect on the death promoting action of cytokines. For example, the growth inhibitory response to cytokines such as TGF-β, was markedly reduced by the inactivation of the *Rb* gene, or the response to IL-6 was enhanced by introducing activated *p53* genes (Pietenpol *et al*., 1990, *Cell,* 61:777-785; Levy *et al*., 1993, *Mol. Cell. Biol*., 13:7942-7952).

Thioredoxin, a small hydrogen carrier protein, has previously been implicated in the IFN-γ-mediated growth arrest of HeLa cells (Deiss, L.P. and Kimchi, A. (1991) Science 234:117-120).

### SUMMARY OF THE INVENTION

In the following, the term *"programmed cell death"* will be used to denote a physiological type of cell death which results from activation of some cellular mechanisms, i.e. death which is controlled by the cell's machinery. Programmed cell death may, for example, be the result of activation of the cell machinery by an external trigger, e.g. a cytokine, which leads to cell death.

The present invention is based on the pioneering finding that inhibition of expression of certain genes counteracts the cytokine-induced cell death. Namely, as long as these genes function normally, cytokine induces cell death; once the expression of said genes is inhibited, the cytokine-induced cell death is inhibited. It follows therefrom that the normal expression product of these genes is involved in programmed cell death, especially in cytokine-induced cell death. In HeLa cells, IFN-γ induces a biphasic process, which comprises an initial cytostatic phase and a subsequent cytotoxic phase (programmed cell death). The novel genes discovered in accordance with the present invention were found to affect only the later, cytotoxic phase. These genes will be referred to herein as *"DAP* (death-associated protein) *genes".* DNA molecules comprising a coding sequence encoding the expression products of the DAP genes, or expression products having a similar biological activity, will be referred to herein at times collectively as *"DAP DNA molecules"*. The expression products of the DAP DNA molecules will be referred to herein at times collectively as "*DAP products"*.

According to one aspect of the present invention, to be referred to herein as "*the death-promoting aspect"*, the above DAP DNA molecules, expression vectors comprising them, or DAP products are used for promoting death of normal or tumor cells. A particular application of the death-promoting aspect is in therapy of diseases or disorders associated with uncontrolled, pathological cell growth, e.g. cancer, psoriasis, and others. The use of DAP DNA molecules in gene therapy or DAP products if produced extracellularly, in accordance with the death-promoting aspect of the invention, may be in conjunction with cytokines, e.g. IFN-γ.

According to another aspect of the invention, to be referred to herein as *"the death-preventing aspect*", agents which prevent the expression of said DAP DNA molecules, or agents which antagonize, inhibit or neutralize the DAP products, are used for protecting cells from programmed cell death. Examples of possible applications of the death preventing aspect of the invention are in prevention of cell death in various degenerative neurological diseases, such as Alzheimer's disease or Parkinson's disease, which are associated with premature death of particular subsets of neurons; prevention of death of T-cells in AIDS patients, which death resembles programmed cell death; prevention of rejection-associated cell death in transplants which is believed to result, at least in part, from programmed cell death; protection of normal cells from the cytotoxic effects of certain anti-cancer therapies; etc.

According to a further aspect of the present invention, referred to herein at times as *"the screening aspect"*, DAP DNA molecules are used in order to screen individuals for predisposition to cancer. In accordance with this aspect, the screening is carried out by comparing the sequence of each of the DAP DNA molecules to each of the respective DAP genes in the individual. The absence of a DAP gene, a partial deletion or any other difference in the sequence that indicates a mutation in an essential region, may result in a loss of function and as a consequence a predisposition for cancer. For screening, preferably a battery of different DAP genes may be used.

The DAP genes seem to play an important role in programmed cell death and the inhibition of their expression or neutralization of their expression products protects the cell from eytokine-promoted cell death. Examples of such genes are those whose sequences are depicted in Figs. 6, 8 and 12, or whose partial sequences are depicted in Figs. 13 and 14. The gene for the known protease cathepsin D, whose sequence is depicted in Fig. 15, is also revealed here for the first time as functioning as a DAP gene.

DAP DNA molecules useful in the death-promoting aspect of the invention may have the nucleic acid sequence of the DAP gene or other sequences which encode a product having a similar biological activity to that of the DAP product. Such DAP molecules include DNA molecules having a sequence other than that of the DAP gene but which, owing to the degenerative nature of the genetic code, encode the same protein or polypeptide as that encoded by the DAP gene.

It is well known that it is possible at times to modify a protein by replacing or deleting certain amino acids which are not essential for a certain biological function, or adding amino acids in a region which is not essential for the protein's biological function, without such modification essentially affecting the biological activity of the protein. Thus, a DAP DNA molecule useful in the death promoting aspect of the invention may also have a modified sequence encoding such a modified protein. The modified sequence has a sequence derived from that of the DAP gene or from that of the above degenerative sequence, in which one or more nucleic acid triplets (in the open reading frame of the sequence), has been added, deleted or replaced, with the protein product encoded thereby retaining the essential biological properties of the DAP product. Furthermore, it is known that at times, fragments of proteins retain the essential biological properties of the parent, unfragmented protein, and accordingly, a DAP DNA molecule useful in the death promoting aspect of the invention may also have a sequence encoding such fragments.

A DNA molecule useful in the death-preventing aspect of the invention may have a sequence which is an antisense sequence to that of the DAP gene, or an antisense sequence to part.of the DAP gene, blocking of which is sufficient to inhibit expression of the DAP gene. The part of the gene can be either the coding or the non-coding part of the DAP gene. The mRNA transcripts of the antisense sequences hybridize to the mRNA transcripts of the DAP gene and interfere with the final protein expression. Another DNA molecule useful in the death preventing aspect of the invention is a DNA molecule coding for a modified DAP product which is capable of inhibiting the activities of the unmodified DAP product in a dominant negative manner, such as catalytically inactive kinase (DAP-kinase) or any other modified protein whose presence in the cell interferes with the normal activity of the native protein, for example by producing faulty hetero dimers comprised of modified and unmodified proteins which are inactive and the like.

DNA molecules useful in the screening aspect of the invention comprise the sequence of a DAP gene or a sequence of a fragment thereof. Additionally, also the above antisense DNA sequences may be used in the screening aspect of the invention.

The present invention thus provides a DNA molecule comprising a sequence selected from the group consisting of:
(a) a gene whose expression is necessary for the mediation of the cytokine-induced programmed cell death;
(b) a DNA sequence encoding the same protein or polypeptide encoded by the gene defined in (a);
(c) a modified DNA sequence of (a) or (b) in which one or more nucleic acid triplets has been added, deleted, or replaced, the protein or polypeptide encoded by the modified DNA sequence mediating the cytokine-induced programmed cell death similarly to the protein or polypeptide encoded by the gene as defined under (a) or (b);
(d) fragments of any of the DNA sequences of (a), (b) or (c), encoding a protein or a polypeptide having said biological activity;
(e) a sequence which is an antisense to the entire or part of the DNA molecule under (a) and capable of inhibiting the expression of said gene; and
(f) a modified DNA sequence of (a) or (b) in which one or more nucleic acid triplets has been added, deleted or replaced, the protein or polypeptide encoded by the modified sequence having dominant negative effect manifested by the ability of said protein or polypeptide to inhibit said cytokine-induced programmed cell death.

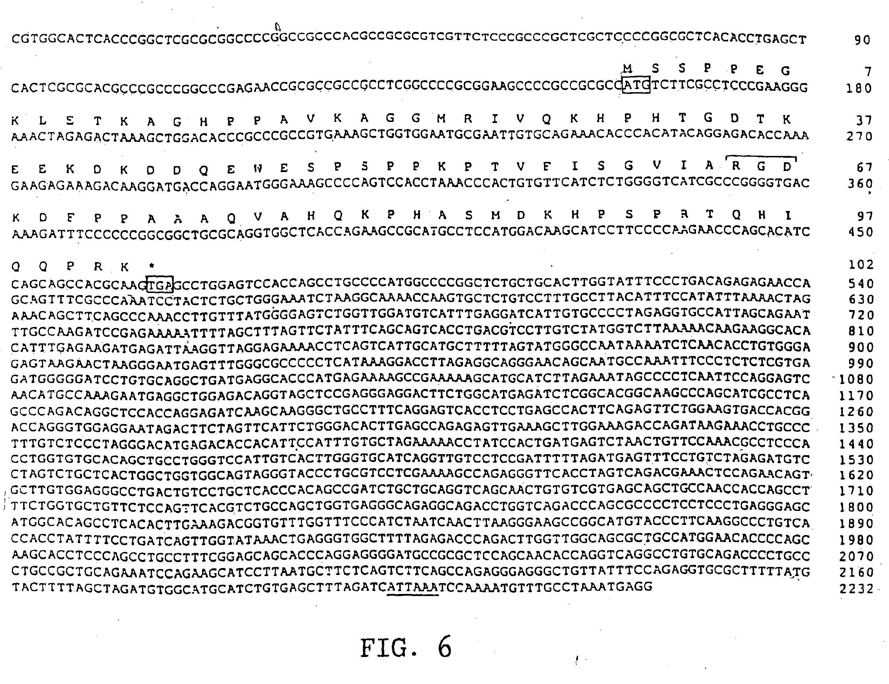

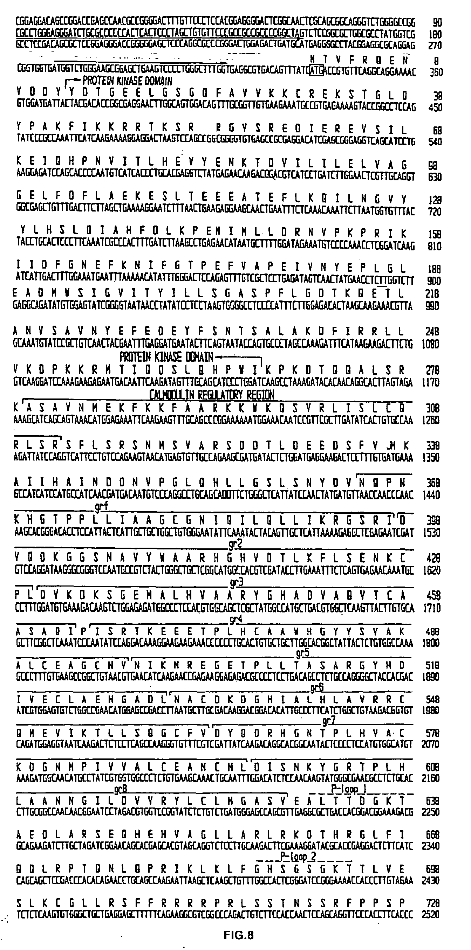

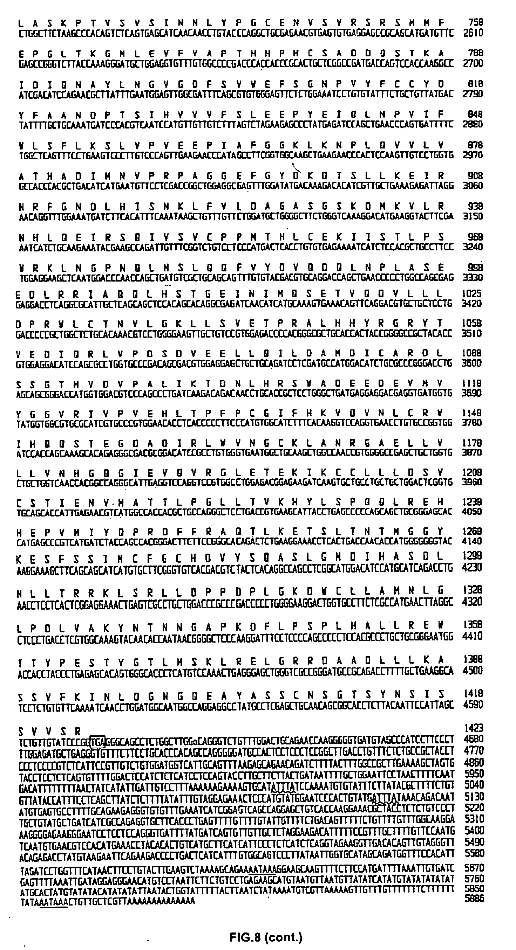

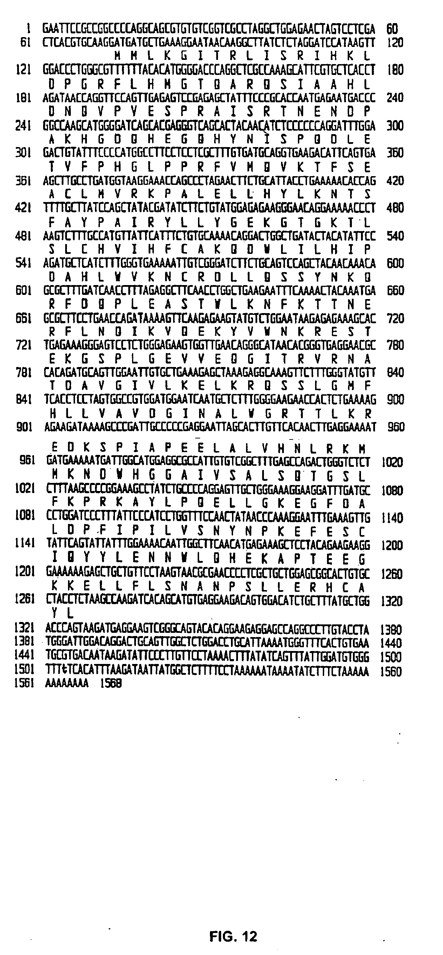

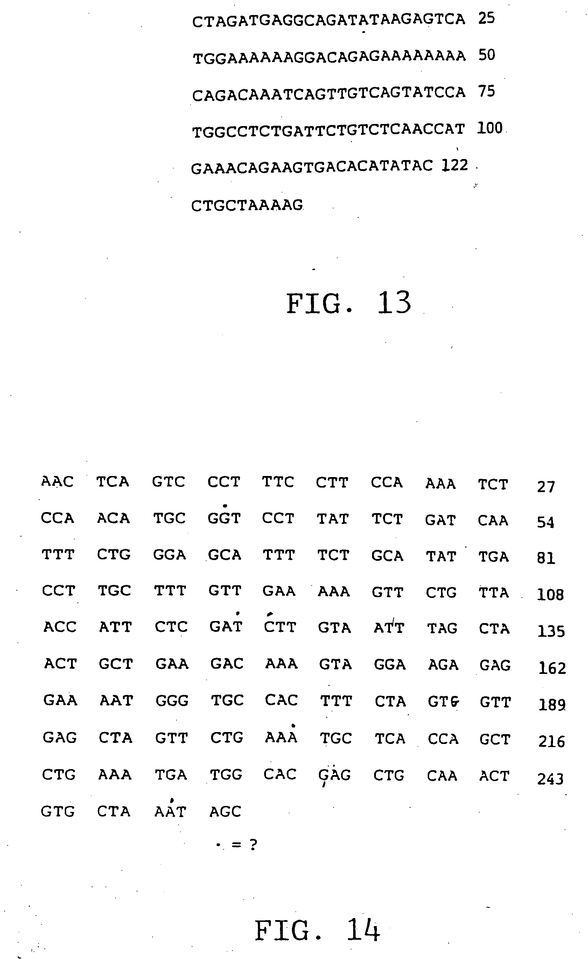

In accordance with a specific embodiment, the present invention provides a DNA molecule comprising a nucleic acid sequence selected from the group consisting of:
(a) a DNA sequence comprising a coding sequence beginning at the nucleic acid triplet at position 160-162 and ending at the triplet 466-468 of the sequence depicted in Fig. 6;
(b) a DNA sequence comprising a coding sequence beginning at nucleic acid triplet at position 287-289 and ending at a triplet at positions 816-818 of the sequence depicted in Fig. 6;
(c) a DNA sequence comprising a coding sequence beginning at nucleic acid triplet at position 337-339 and ending at the triplet at position 4603-4605 of the sequence depicted in Fig. 8;
(d) a DNA sequence comprising a coding sequence beginning at position 74-76 and ending at position 1268-1270 of the sequence depicted in Fig. 12;
(e) a DNA sequence comprising a sequence depicted in Fig. 13;
(f) a DNA sequence comprising a sequence depicted in Fig. 14;
(g) a DNA sequence encoding the same protein or polypeptide encoded by any one of the DNA sequences of (a) - (f);
(h) a DNA sequence as in (a) - (g) in which one or more nucleic acid triplets has been added, deleted or replaced, the protein or polypeptide encoded by the sequence having essentially the same biological activity as that encoded by any one of the DNA sequences of (a) - (g), respectively;
(i) fragments of any one of the DNA sequences of (a) - (h), encoding a protein or polypeptide retaining a biological activity present in the protein or polypeptide encoded by any one of the DNA sequences under (a)-(h), respectively;
(j) a sequence which is an antisense to the entire or pan of any one the sequences under (a) - (f) or of the cathepsin D gene and capable of inhibiting the expression of said sequence; and
(k) a modified DNA sequence of any one of the sequences (a) - (f) in which one or more nucleic acid triplets has been added, deleted or replaced, the protein or polypeptide encoded by the modified sequence having dominant negative effect and being capable of inhibiting the function of the protein or polypeptide encoded by any one of the sequences (a) - (f), respectively.

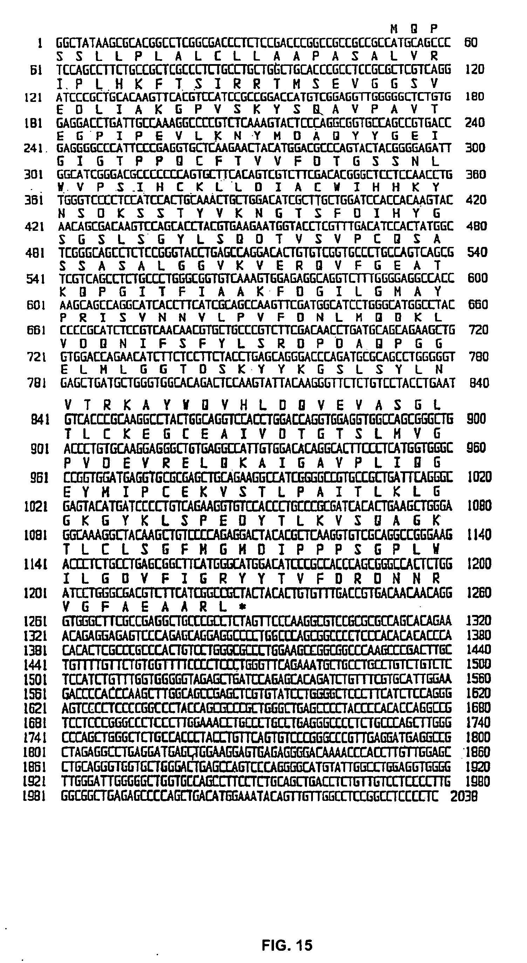

The preferred antisense sequences as defined in (j) above are those to the sequences beginning at position 1000 and ending at position 1320 of the DAP-1 gene in Fig. 6, 3781-4148 of the DAP-2 gene in Fig. 8, 74-1270 of the DAP-3 gene in Fig. 12, and 1203-1573 of the cathepsin D gene in Fig. 15.

The present invention also provides a vector comprising any of the above DNA molecules, the vector comprising also sequences required for maintaining and replicating it in a host cell. Vectors in accordance with the present invention may be transfer vectors for propagating and replicating the DNA sequences in a host cell or may be expression vectors comprising also sequences required for translation of said DNA sequences into an mRNA. Examples of such expression vectors are plasmids, e.g. episomes or viruses. Examples of episomes are those constructed by using the vehicles pTKO1, pTKO2 and pTKO3 (Deiss and Kimchi, *supra*).

The present invention also provides a DAP product which is a protein or polypeptide encoded by a DNA molecule of the invention, with the exception of the DNA molecules having an antisense sequence, or such a protein or polypeptide which has been chemically modified, for example, by methylation, glycosylation, etc. An example of a DAP product is that having the amino acid sequence depicted in Figs. 6, 8 and 12. The DAP product is useful in the death-promoting aspect of the present invention. In accordance with this aspect, the protein may be administered to patients, in particular, to cancer patients, which administration may cause death of the transformed cells.

The present invention further provides agents which inhibit, antagonize or neutralize the DAP product, which are useful in the death-preventing aspect of the invention. Such agents are for example, antibodies directed against the DAP product; inhibitors or antagonists of the DAP product which are able to counteract their effect and prevent the death-promoting activity of the DAP product.

The present invention further provides a pharmaceutical composition comprising a pharmaceutically acceptable carrier and an active agent being selected from the group consisting of: (i) an expression vector comprising a DNA molecule of the invention or a DNA molecule coding for cathepsin D; (ii) a DAP product of the invention or cathepsin D, and (iii) an antibody, inhibitor or antagonist to the DAP product. The pharmaceutical composition of the present invention may also comprise means for targeting said active agent to the desired cell or tissue. Depending on the nature of the active agent, the composition is useful either in accordance with the death-promoting or the death-preventing aspect of the invention. In accordance with the death-promoting aspect of the invention, the pharmaceutical composition may also comprise a cytokine, e.g. IFN-γ, in combination with a suitable DAP product, or with an expression vector comprising a suitable DAP molecule.

Further provided by the present invention is a method of treatment comprising administering said active agent to an individual. Similarly as in the pharmaceutical composition, depending on the nature of said active agent, the method is practisable in either the death-promoting aspect of the invention or the death-preventing aspect of the invention. In the death-promoting aspect of the invention, said active agent may be administered in conjunction with a cytokine, e.g. IFN-γ.

In accordance with the screening aspect of the invention, there is provided a method for detecting the absence of the DAP gene, a partial deletion or a mutation (i.e. point mutation, deletion or any other mutation) in the DAP genes of an individual, comprising probing genomic DNA or cDNA from the individual with a DNA probe or a multitude of DNA probes having a complete or partial sequence of the DAP genes or having a sequence which is an antisense to the complete or partial sequence of the DAP gene. A particular application of the screening aspect of the invention is in the screening for individuals having a predisposition to cancer, an absence of the gene or a detected mutation or deletion indicating that the individual has such predisposition. The method in accordance with the screening aspect typically comprises the following steps:
(a) obtaining a sample of either genomic DNA from cells of the individual or cDNA produced from mRNA of said cells;
(b) adding one or more DNA probes each of said probes comprising a complete or partial sequence of a DAP gene, or a sequence which is an antisense sequence to the complete or partial sequence of the DAP gene;
(c) providing conditions for hybridization between the DNA probe or probes and the DNA of said sample;
(d) on the basis of the hybridization determining whether the DAP gene is absent or there is a match between the sequence of the DNA probe or probes and a sequence in the DNA of said sample or a mismatch, a mismatch indicating a deletion or a mutation in the genomic DNA and a predisposition to cancer in the tested individual.

A specific embodiment of the screening aspect of the invention involves use of a complete or partial sequence of that shown in Figs. 6, 8, 12-14 or 15, or an antisense of the complete or partial sequence in Figs. 6, 8, 12-14 or 15.

The mutation in the DAP gene indicating a possible predisposition to cancer can also be detected by the aid of appropriate antibodies which are able to distinguish between a mutated and non-functional and a normal functional DAP gene product.

### DESCRIPTION OF THE DRAWINGS

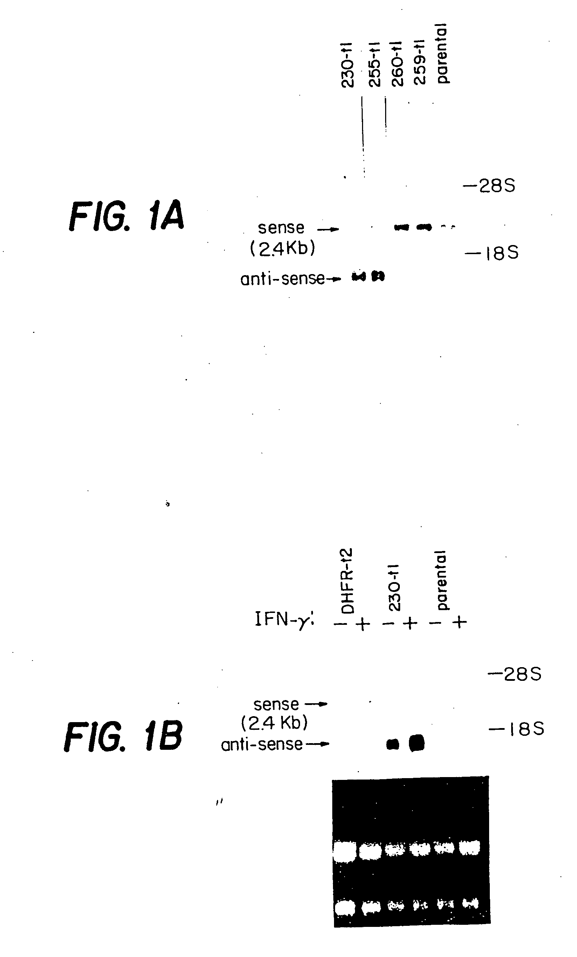

**Figs. 1 A-D** show RNA and protein expression of the DAP-1 gene, wherein:
   **Fig. 1(A)** shows a Northern blot analysis of sense and antisense mRNA obtained from HeLa cells transfected with the constructs 230, 255, 260, 259 and control cells (parental cells) and probed by labeled cDNA fragments from construct 230. Total RNA was prepared from HeLa cells either before (parental) or after transfection with pTKO1 constructs #230 or #255 (group 1), #260 (group 5) and #259 (group 3) designated 230-t1, 255-t1, 260-t1 and 259-t1, respectively. Twenty µg RNA were processed on Northern blots and DNA fragment #230 was used as a probe. The arrows point to the position of sense and antisense RNAs.
   **Fig. 1(B)** shows a Northern blot analysis of sense and antisense mRNA obtained from HeLa cells transfected with control construct (DHFR-t2), 230 construct or control cells (parental) cells treated with (+) or without (-) 750 U/ml of IFN-γ for 24 h. The RNA was extracted from the indicated HeLa cells which were grown for 4 days in the absence (-) or presence (+) of IFN-γ (750 U/ml). The Northern blot containing 20 µg RNA samples was hybridized with the cDNA insert of λ1 phage. The Ethidium Bromide staining of the mRNA samples is shown.

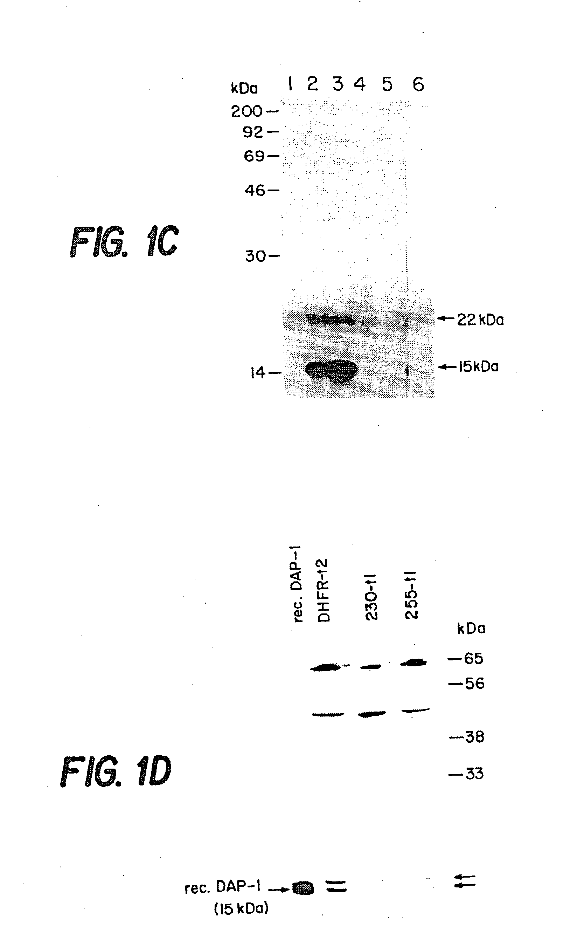

   **Fig. 1(C)** shows an SDS polyacrylamide electrophoresis gel of the expressed protein product of DAP-1 cDNA translated *in vitro* in a reticulocyte lysate preparation. *In vitro* translation of RNA (0.5 µg) transcribed from the λ1 cDNA (lane 2) and from the subclones p6, p4, p5 and p8 are shown in lanes 3-6, respectively. Lane 1 corresponds to the background obtained in the absence of RNA administration to the reticulocyte lysates. The labeled proteins were fractionated on 12% SDS polyacrylamide gels. The position of the radioactive molecular weight markers (Amersham) is marked. The two translated proteins, the major 15kDa and minor 22kDa proteins, are indicated by arrows.
   **Fig. 1(D)** shows an immunoblot analysis of recombinant and cellular 15kDa DAP-1 protein. Bacterially produced DAP-1 protein (300 ng) and the indicated HeLa cell extracts (350 µg) were fractionated on SDS polyacrylamide gels (12%), blotted to nitrocellulose and reacted with affinity purified antibodies generated against the 15kDa DAP-1. The cells were treated with IFN-γ (750 U/ml) for 4 days before their extraction. The two arrows point to the position of the cellular DAP-1 protein. The antibodies also recognize two non-relevant bands of 60 and 45 kDa that are not modulated by the antisense RNA expression. Quantitation of the reduction in DAP-1 protein was done by densitometric analysis. The calibration of the protein content in each slot was done by referring to the signals of the non-relevant bands. The prestained protein markers (Sigma) are marked.

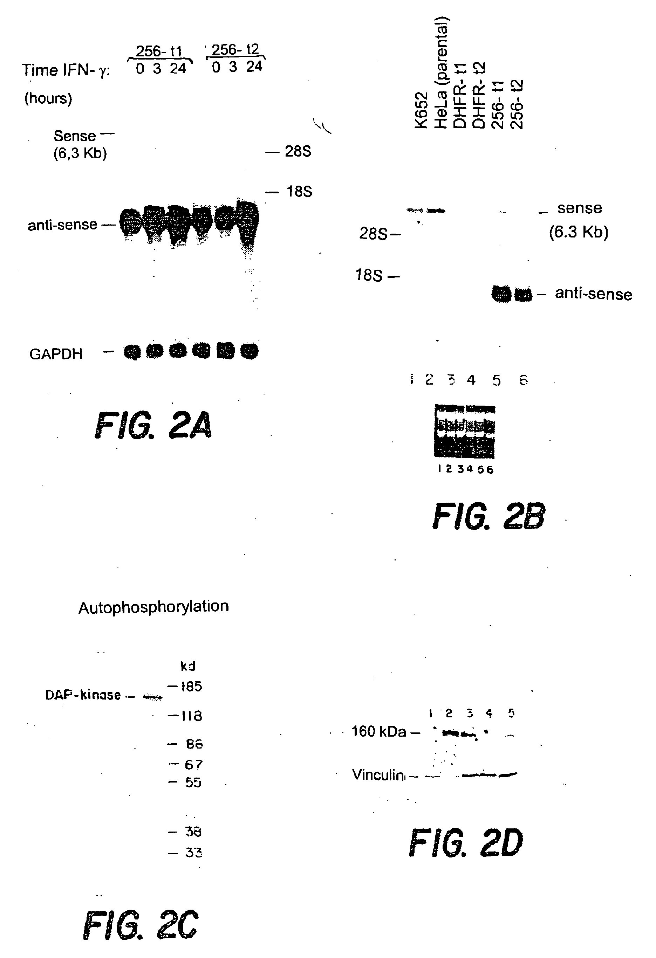

**Figs. 2 A-D** show RNA and protein expression of the DAP-2 gene, wherein:
   **Fig. 2(A)** shows a Northern blot analysis of sense and antisense mRNA obtained from two clones of HeLa cells transfected with the control constructs (DHFR-t1 and DHFR-t2) and two clones of cells transfected with the 256 construct (t1 and t2). Total RNA was prepared from the 256-t1 and 256-t2 HeLa cell transfectants either before (0 hours) or at 3 and 24 hours after treatment with IFN-γ (750 U/ml) and 20 µg samples were processed on Northern blots. Fragment #256 was used as a probe. The position of the sense and antisense mRNAs is indicated. The GAPDH mRNA levels were used for the calibration of the RNA amounts in each blot.
   In **Fig. 2(B)** the blot consists of total RNA (20 µg) from K562 cells, parental HeLa cells, the two DHFR-transfected HeLa cell populations and the two HeLa cell populations that were transfected with the pTKO1-256. The blot was hybridized with the cDNA insert of λ29. The Ethidium Bromide staining of the RNA samples is shown.
   **Fig. 2(C)** shows an *in vitro* phosphorylation assay. Cell lysates were prepared from COS-7 cells either before (lane 1) or after transfection with the PECE-FLAG expression vector that carries the coding region of the λ29 cDNA (lane 2). Samples of 400 µg were immunoprecipitated with anti-FLAG™ (M2) monoclonal antibodies (IBI) and subjected to phosphorylation assays.
   **Fig. 2(D)** shows immunoblot analysis of recombinant and cellular DAP-2 protein. The COS-7 cells were transiently transfected with the PECE-FLAG-DAP-2 expression vector. Samples of cell lysates, 100 µg from COS-7 cells and 400 µg from HeLa cells, were fractionated on SDS polyacrylamide gels (7.5%), immunoblotted and reacted with affinity purified polyclonal antibodies raised against the N-terminal DAP-2 peptide. In the lower panel the blot was reacted with monoclonal antibodies against vinculin (Sigma Immunochemicals). Lanes: 1, non-transfected COS-1 cells; 2, transfected COS-1 cells; 3, DHFR-t1 cells; 4, 256-t1 cells; 5, 256-t2 cells. In lane 2 the same 160 kDa protein was also detected with anti-FLAG™ (M2) monoclonal antibodies (IBI) (not shown).

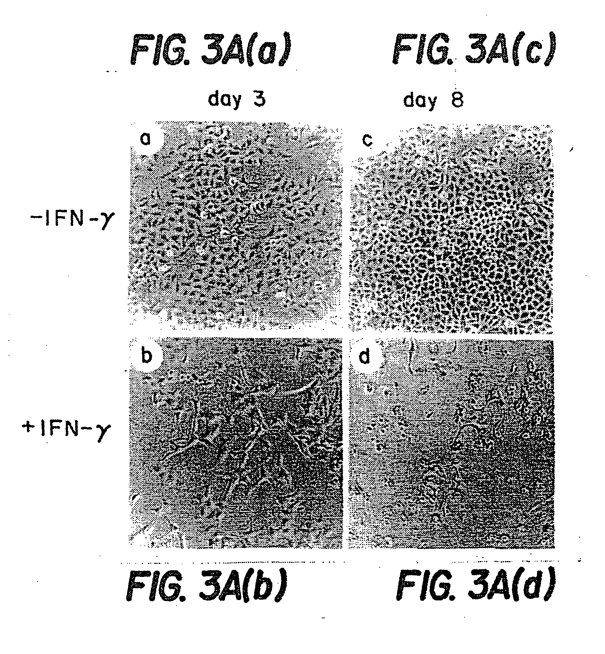

**Figs. 3 A-C** show morphological features of the cytostatic and cytotoxic responses to IFN-γ in HeLa cells. All cultures were seeded at an initial density of 10,000 cells per cm².
**Fig. 3(A)** shows light microscopy of HeLa cells transfected with pTKO1-DHFR construct (DHFR-t1 cells), on days 3 and 8 of culturing in the absence (a,c) or the presence (b,d) of IFN-γ (750 U/ml). (Magnification x 400). Note the absence of refractile mitotic cells during the cytostatic phase of responses to IFN-γ (in b) and the appearance of round cells that were detached from the substratum during the killing phase (in d).

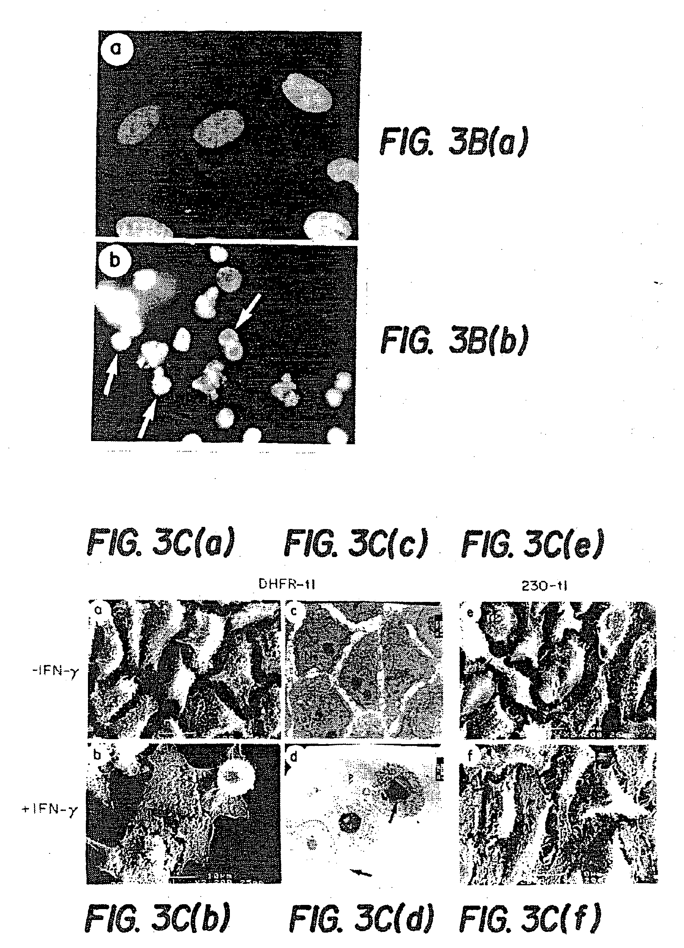

**Fig. 3(B)** shows staining of DNA with DAPI; a. DHFR-t1 non-treated cells removed by trypsinization and mounted on glass slides. b. Detached DHFR-t1 cells collected 7 days after IFN-γ treatment. Nuclei with condensed or fragmented chromatin are indicated by arrows. (Magnification x 1000).
**Fig. 3(C)** shows scanning and transmission electron micrographs of cells transfected with the control construct DHFR-t1 and the 230-t1 construct. DHFR-t1 HeLa cell populations (a-d) and the 230-t1 antisense transfected cells (e and f), were cultured either in the absence (a, c, e) or in the presence (b, d, f) of IFN-γ (750 U/ml). (a,b,e,f), scanning electron micrographs were taken after 7 days using GSM 6400 SEM (Jeol). Bars=10 mm (x2200 magnitude in all the four samples). (c and d), transmission electron micrographs taken after 7 days using TEM (Philips 410) at a magnitude of x2800. The condensed nuclei and the surface blebs are indicated by arrows.

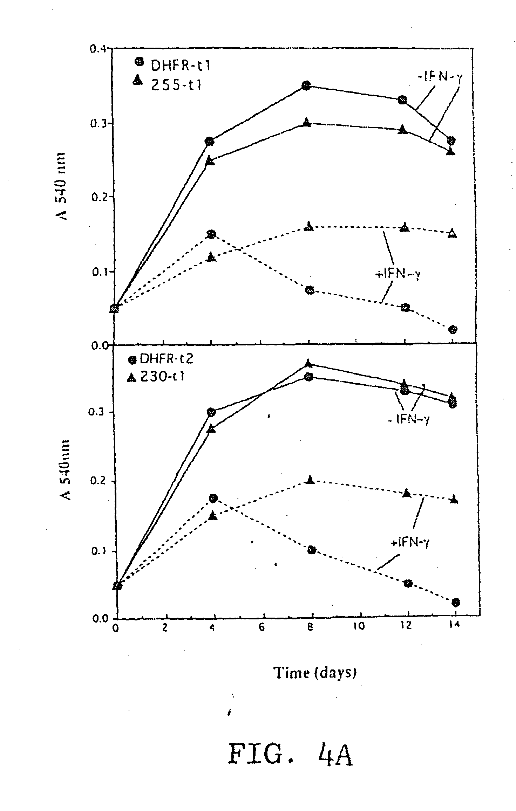

**Figs. 4 A-C** show that the antisense RNA expression from plasmids of groups 1 and 2 reduces the susceptibility of HeLa cells to the killing effects of IFN-γ but has no effect on early IFN-γ signalling.

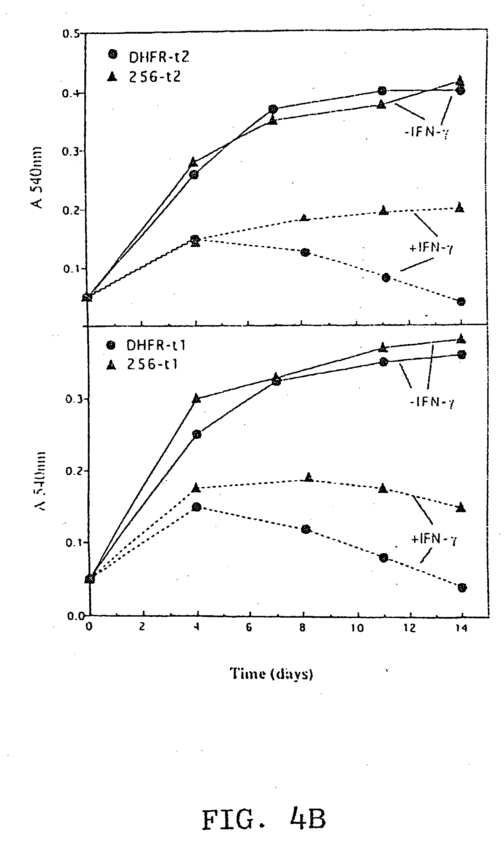

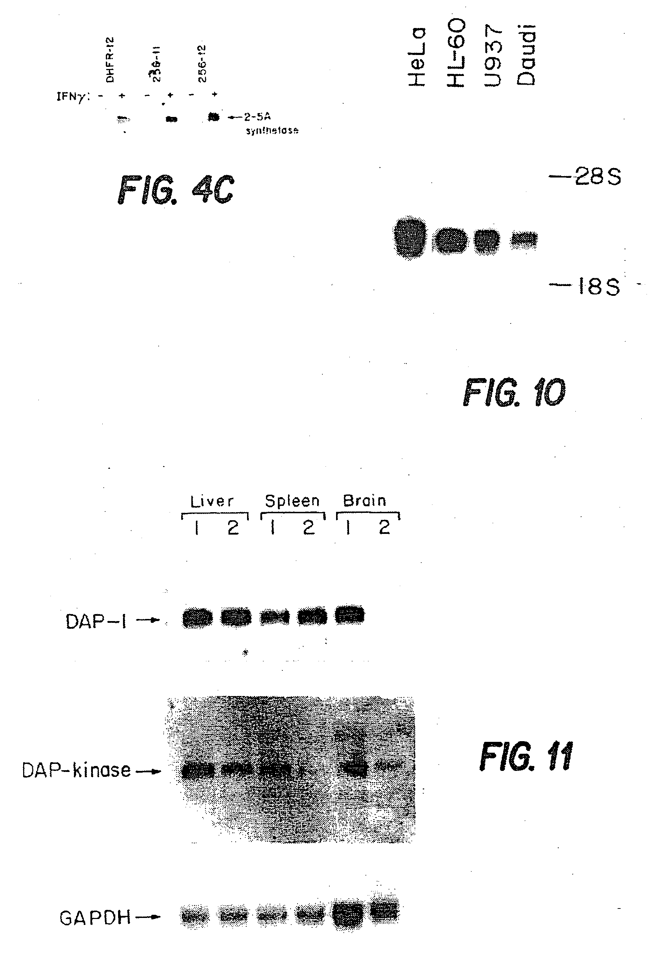

**Figs. 4 (A-B)** show the number of viable cells as determined by light absorption at 540 nm, as a function of time; the cells being transfected either with the control construct DHFR-t1 (• - 1(A) and 1(B)); the 255 or 230 construct (▲ - 1(A)) or with two clones t1 and t2 of the 256 construct (▲ - 1(B)). The results are shown both for cell growth with (+) and without (-) administration of 750 U/ml of IFN-γ. Each point is the average of a quadruplicate determination with a SD that ranged between 2-5%.
**Fig. 4(C)** shows a Northern blot analysis of 2-5A synthetase gene induction. The indicated HeLa cell transfectants were incubated for 24 hours in the presence (+) or absence (-) of IFN-γ (750 U/ml). Twenty mg of total RNA were analyzed. The cDNA of the 2-5A synthetase was used as probe.

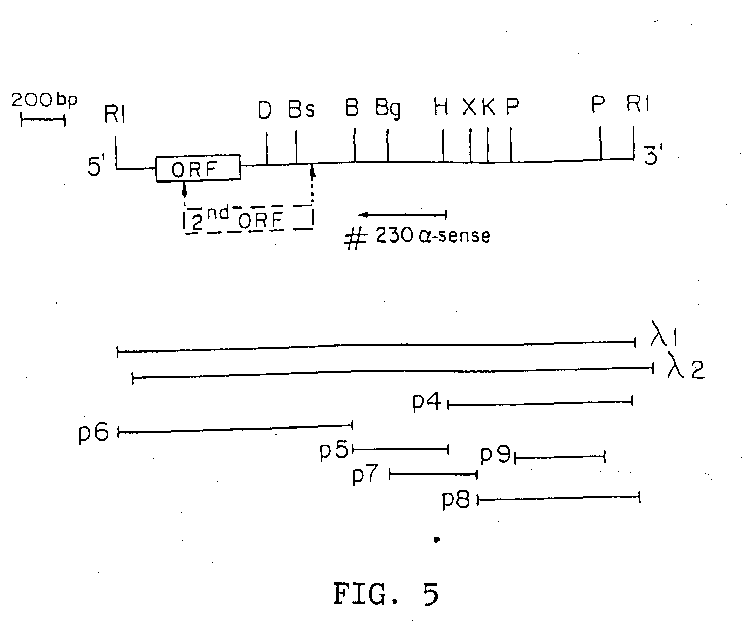

**Fig. 5** shows the restriction map of the λ1 cDNA clone that carries the DAP-1 cDNA.
**Fig. 6** shows the DNA sequence and predicted amino acid sequence of DAP-1.

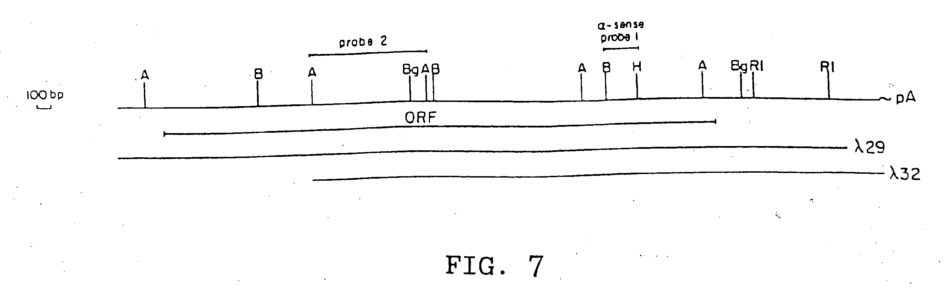

**Fig. 7** shows the restriction map of the λ29 cDNA clone, that carries the DAP-2 cDNA.
**Fig. 8** shows the DNA sequence and predicted amino acid sequence of DAP-2.

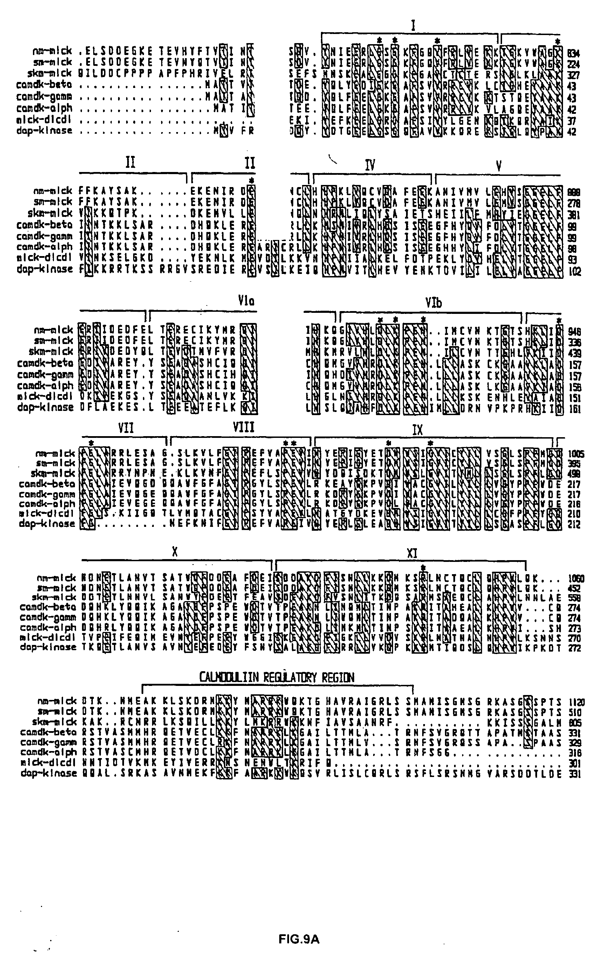

**Figs. 9 A-C** show DAP-2 sequence homologies to other serine/threonine kinases and alignment of the ankyrin repeats of DAP-2, wherein:
   In **Fig. 9(A)** the protein kinase domain sequences of the DAP-2 are aligned with the corresponding domains of other calmodulin-dependent kinases. The kinase subdomain structure (numbered I-XI) and the region implicated in calmodulin recognition and binding (designated as calmodulin regulatory region) are indicated. The obligatory conserved amino acids within the kinase domain are labeled with asterisks. Numbers at the right mark positions relative to the N-terminus of primary translational products of each kinase. Solid background indicates identical amino acids within the compared kinases. Stippled background indicates positions where the amino acids are not identical but similar nm-mlck - non-muscle myosin light chain kinase (chicken); sm-mlck - smooth muscle myosin light chain kinase (chicken); skm-mlck - skeletal muscle myosin light chain kinase (rat); camdk-alph,-beta,-gamm - calcium/calmodulin dependent protein kinase II - α-, β- and γ- subunits, respectively; mlck-dicdi - dictyostelium discoidium (slime mold) myosin light chain kinase.

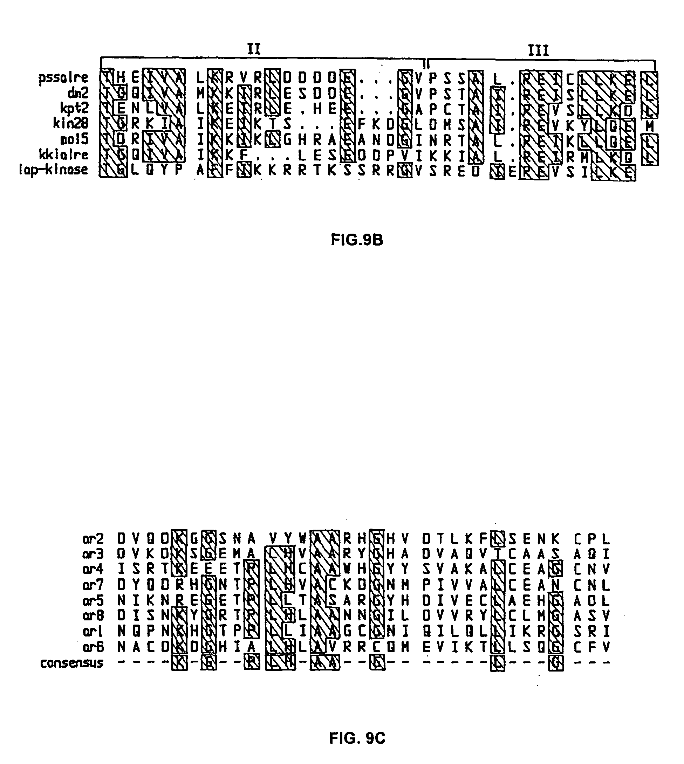

   **Fig. 9(B)** shows alignment of kinase subdomains II and III of DAP-2 and the corresponding domains of different cell cycle dependent kinases. dm2 - Drosophila CDC2 homologue; pssalre - Human serine/threonine kinase PSSALRE; kpt2 - Human serine/threonine protein kinase PCTAIRE-2; kin28 - yeast (*S.cerevisiae*) putative protein kinase; mo15 - Xenopus protein kinase related to cdc2 that is a negative regulator of meiotic maturation; kkialre - human serine/threonine protein kinase KKIALRE.
   **Fig. 9(C)** shows alignment of DAP-2 ankyrin repeats. Solid background indicates identical amino acids. A consensus sequence of the DAP-2 ankyrin repeats is shown at the bottom. The position of each individual repeat along the cDNA is illustrated in Fig. 9(B). at 1-8, ankyrin repeats.
**Fig. 10** shows Northern blot analysis of mRNA obtained from several hematopoietic cells probed with labeled DAP-1 cDNA.
**Fig. 11** shows Northern blot analysis of mRNA obtained from liver, spleen or brain of normal embryos (2) and embryos with Down Syndrome (1) both probed with the labeled cDNA or DAF-1 or DAP-2. In order to evaluate levels of total mRNA, GAPDH was used (bonom).
**Fig. 12** shows the DNA sequence and predicted amino acid sequence of DAP-3.
**Fig. 13** shows a partial DNA sequence of DAP-4.
**Fig. 14** shows a partial DNA sequence of DAP-5.
**Fig. 15** shows the DNA sequence and amino acid sequence of cathepsin D.

### DETAILED DESCRIPTION OF THE INVENTION

### I. Isolation of antisense cDNA's that protect cells from the cytotoxic effects of IFN-γ

### (A) Experimental procedure

### (A₁) Obtaining cDNA clones

A cDNA library (100µg DNA) was generated from a mixture of mRNA's harvested before and at 1, 2, 4, 12, 24 and 48 hours after treatment of HeLa cells with IFN-γ (200 U/ml). It was cloned in antisense orientation into the EBV-based pTKO1 expression vector, as previously described in detail (Deiss and Kimchi, *supra*). The resulting expression library of about 10⁵ independent clones was introduced into 8X10⁶ HeLa cells (10⁶ cells per 9 cm plate) by the calcium phosphate transfection technique. In order to determine the efficacy of transfection, a fraction of the transfectants was selected with hygromycin B (200 µg/ml, Calbiochem). The resulted efficacy was around 5%. In parallel, the majority of the transfected cells were plated at a cell density of 1500 cells per cm² and were selected with both hygromycin B (200 µg/ml) and IFN-γ (750 U/ml). Selective media was changed every 3-4 days. After 28 days the cells that survived and/or grew in the presence of IFN-γ were expanded for 2 weeks and pooled. The extrachromosal DNA was obtained according to the method of Hirt (Hirt, B. (1967) *J. Mol. Biol*., **26**:365), cleaved with the restriction enzyme Dpnl and introduced into *Escherichia coli* HB101 host cells. The cleavage with DpnI ensured that only episomal DNA that have replicated in HeLa cells was transfected into bacteria.

A few bacterial clones were obtained by the above procedure which included DNA antisense sequences, some of which were able to protect the cells from the death-promoting effects of IFN-γ.

### (A₂) Classification of the antisense cDNA clones

Plasmid DNAs were prepared from 10 individual bacterial clones. PCR amplified cDNA inserts were generated from each plasmid using specific primers that correspond to the immediate flanking sequence of the cDNA insertion sites in the pTKO1 vector. The size of the cDNA inserts ranged between 300 to 800 bp. The PCR fragments were used as labeled probes to search on Southern blots for possible cross hybridization between some of the rescued antisense cDNA clones.

### (B) Results

### (B₁) Classification of Clones

The above 10 cDNA clones were classified into six distinct nonoverlapping groups, some constituting several members (clones) and some constituting of a single member. Those clones relevant for the present invention are shown in the following Table 1:

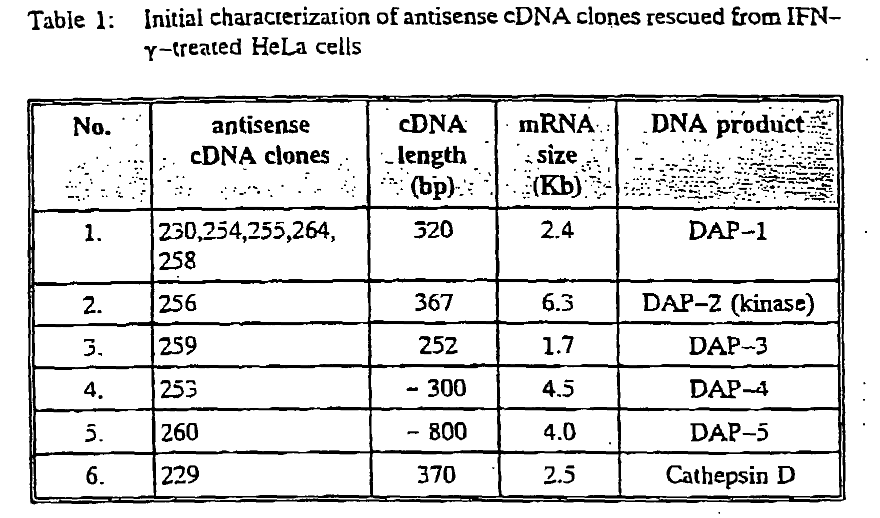

Inserts 230, 254, 255, 264 and 258 of group 1 seemed to be completely identical to one another. The PCR fragments were sequenced and the results were compared with sequences present in the EMBA nucleic acid database. All inserts of groups 1 through 5 were found to be novel.

### (B₂) Detection of mRNA

The DNA fragments thus obtained were used to detect and determine the expression level in HeLa cells of mRNA which hybridized to these fragments. 20 µg of total RNA from the parental HeLa cells were fractionated on gels, blotted and reacted with the different probes. Each probe recognized a single mRNA transcript of a different size (Table 1). Expression levels of mRNA's reactive with group 2 were low while those reactive with group 1 were relatively high.

### II. Second transfection by isolated antisense cDNA

### Levels of expression of antisense RNA in secondary transfectants

### (A) Experimental procedure

To ensure that the above isolated antisense cDNA's are sufficient in order to protect cells from the death promoting effects of IFN-γ, subconfluent monolayers of HeLa cells were transfected with 40 µg DNA of the individual rescued pTKO1 plasmids (in duplicates) and subjected to the single selection of hygromycin B. Pools of approximately 10⁴ hygromycin resistant clones were generated from each transfection and were kept as 6 duplicates of stable polyclonal populations. The sensitivities of the above clones to an application of IFN-γ was then determined.

The vector pTKO1-DHFR (Deiss and Kimchi, *supra*) which carried a non-relevant construct served as control. The control vector was introduced in parallel into HeLa cells and produced two independent polyclonal population of stable transfectants designated DHFR-t1 and t2.

The double stranded cDNA fragments from construct 230 and 256 (from groups 1 and 2, respectively) were used as probes in Northern blot analysis in order to detect mRNA transcripts both in non-transfected and transfected HeLa cells. These two specific cDNA inserts were labelled by commonly used commercial labelling kits. They were subcloned into Bluescript™ vectors (Stratagene, USA) to facilitate both the preparation of the cDNA inserts and the production of single stranded RNA probes therefrom. (B) Results

### Constructs 230 (group 1)

As can be seen in Fig. 1A the cDNA insert in this construct hybridized to a single endogenous 2.4 Kb mRNA transcript, both in non-transfected and transfected HeLA cells. In stable transfectants containing the antisense constructs of clones 230 and 255, an additional composite antisense transcript was detected by this 230 probe. It consisted of 320 bases of the original cDNA insert and 800 additional bases of sequences derived from the expression cassette (SV40 early promoter together with sequences till the polyadenylation signal). One of the RNA labeled strands produced by the Bluescript™ vector hybridized exclusively to the endogenous 2.4 Kb mRNA while the complementary strand hybridized only to the 1.1 Kb RNA confirming that the latter is indeed an antisense mRNA (data not shown).

The amount of the antisense RNA in clones 230 and 255 exceeded the sense mRNA levels by 3 to 6 fold (Figs. 1A, 1B). After IFN-γ treatment the level of antisense expression was further elevated due to the presence of IFN-γ-stimulated response element (ISRE) in the pTKO1 vector (Deiss and Kimchi, *supra*), thus leading to 15 fold excess of antisense over sense transcripts (Fig. 1B). The endogenous 2.4 Kb mRNA level was neither modulated by IFN-γ, nor influenced by the high antisense expression.

### Construct 256 (group 2)

As can be seen in Figs. 2A and 2B, the construct of the 256 clone (367 bp in size) hybridized on Northern blots to a single endogenous 6.3 Kb mRNA transcript which was expressed in all tested cells at relatively low levels. In the 256-t1 and t2 transfected cells it also hybridized to a composite 1.2 Kb RNA that consisted of 367 bases of the cDNA insert and 800 bases of sequences derived from the expression cassette in the vector (Fig. 2). The antisense orientation of fragment #256 in the pTKO1 vector was confirmed upon sequencing of the sense cDNA clone (Fig. 7). The amount of the antisense RNA expressed from pTKO-1 plasmid #256 in non-treated HeLa cells exceeded the sense mRNA levels by more than 100 fold. Moreover, due to the presence of IFN-stimulated response element (ISRE) in the pTKO1 vector, the levels of antisense mRNA expression were further elevated after IFN-γ treatment (Fig. 3).

### III. Response of cells transfected with antisense cDNAs to IFN-γ

### (A) Experimental procedure

The HeLa polyclonal population transfected with the individual antisense cDNAs were cultured in the presence of both hygromycin B and IFN-γ (750 U/ml). Growth and viability parameters were examined: (1) under the light microscope, (2) by electron microscopy, and (3) by DAPI staining (0.5 µg/ml; Sigma). For more detailed quantitation, a neutral red uptake assay was performed: the different polyclonal HeLa cell populations were cultivated in 96-well microtiter plates at subconfluent cell densities and then treated with IFN-γ (750 U/ml) or left untreated. All the cells were continuously maintained in a hygromycin B-containing medium to select for transfected cells. The two DHFR-transfected HeLa cell populations (t1, t2), prepared as described above, served as control cultures that display the typical growth sensitivity curves to IFN-γ. The examined antisense cDNA transfected cells were the 230-t1, 255-t1 (group 1) and 256-t1, 256-t2 (group 2). Viable cells were stained with neutral-red and the dye uptake was quantified by measuring O.D. at 540 nm in quadruplicates during the 14 days of the experiment.

### (B) Results

The microscopic examination of parental and control DHFR-transfected HeLa cells revealed that IFN-γ triggered a biphasic pattern of responses. The cells stopped proliferating during the first four days of IFN-γ treatment but still remained viable (in trypan-blue exclusion tests) and displayed a flattened morphology characteristic of the cystostatic responses to IFN-γ (Fig. 3A, b). The reduction in the proliferation rate during this period was also measured by a sharp decline (by more than 90%) in the thymidine uptake into DNA (not shown). This type of IFN-γ-induced proliferation arrest was then followed by massive cell death that occurred in a non-synchronous fashion over a period of an additional 10 days. The cells gradually reduced their size, rounded up and detached from the plates (Figs. 3A, d). Staining of DNA with DAPI after detachment of cells from the substratum revealed gross changes in the nuclear morphology characteristic of programmed cell death. This included nuclear pyknosis, chromatin condensation, sometimes detected preferentially at the nuclear periphery, and chromatin segmentation (Fig. 3B, b). Transmission electron micrographs of the IFN-γ-treated cells prior to their detachment revealed other morphological changes including the disappearance of surface microvilli, surface blebbing, budding off cytoplasmic projections and cytoplasmic disintegration, in addition to the nuclear pyknosis and chromatin condensation (details shown in Fig. 3C, d). The antisense RNA expression from pTKO-1 plasmid of group 1 reduced the susceptibility of the cells to the killing effects of IFN-γ: more cells survived on the plates and the abovementioned death associated morphological changes appeared at much lower frequency (compare the scanning electron micrographs of the IFN-γ-treated DHFR-transfected cells in Fig. 3C, b to the IFN-γ-treated 230-t1 cells in Fig. 3C, f). Similar microscopic observations, showing protection from the IFN-γ-induced cell death, were also made with respect to three other clones from the aforementioned groups of antisense cDNAs, i.e. 2, 3, and 7 (not shown).

A neutral-red uptake assay was then performed to determine more accurately, on a quantitative basis, both the typical biphasic responses of control cultures to IFN-γ and the reduced susceptibility of the antisense expressing cultures to the IFN-γ-induced cell death. The two DHFR-transfected HeLa cell populations (t1, t2) served as the control cultures in this assay and the antisense cDNA transfected cells examined were the 230-t1, 255-t1 (group 1) (Fig. 4A) and 256-t1, 256-t2 (group 2) (Fig. 4B). In the absence of IFN-γ, all the transfected HeLa cells behaved the same and displayed practically identical growth curves suggesting that the antisense RNA expression had no effects on the normal growth of cells. Another feature that was not changed by the antisense RNA expression was the extent of the cytostatic responses to IFN-γ. As shown in Figs. 4A and 4B, IFN-γ has similarly reduced the proliferation rate of all the transfected cultures and they all displayed the same extent of reduction in the neutral-red dye uptake during the first 4 days (before cell death starts to be microscopically evident). After 4 days of treatment the picture changed drastically. While almost all control cells died during the subsequent days of IFN-γ treatment leading to minimal values of the neutral-red dye uptake on day 14, a significant fraction of cells that expressed antisense RNA survived in the presence of IFN-γ, as reflected by the sustained values of the dye uptake. The resistance to the IFN-γ-induced cell killing was very similar in all the four tested cultures that expressed the two different antisense RNAs (Figs. 4A, 4B). These data indicate that expression of antisense RNA from groups 1 and 2 protects the HeLa cells exclusively from the IFN-γ-induced cell death and not from its cytostatic action. It is noteworthy that the antisense RNA expression did not affect the early biochemical steps in the signaling of IFN-γ as deduced from the normal mRNA induction by IFN-γ of the 2-5A synthetase gene in these transfected cells (Fig. 4C). Altogether, it is concluded that among all criteria tested only the death inducing effects of IFN-γ were interrupted by the antisense RNA expression.

### IV. Responses of cells transfected with antisense constructs to necrotic cell death

It became interesting at this stage to check whether the antisense RNA expression can also protect the HeLa cells from a necrotic type of cell death. For this, the effect of TNF-α added in combination with cycloheximide (CHX) was examined in the various HeLa cell populations. Unlike the effect of IFN-γ, the cell death that was induced by TNF-α + CHX in HeLa cells was very rapid (50% killing after 3 hours) and displayed typical features of necrosis such as swelling of the cells before their lysis. As shown in Table 2, while the antisense RNA expression from groups 1 and 2 protected the cells from the IFN-γ-induced cell killing, there was no protection from the TNF-α-induced necrotic cell death. All the examined HeLa cell transfectants were killed by the TNF + CHX combination with similar time kinetics and at the same efficiency. Northern blot analysis demonstrated that the levels of the antisense mRNA transcripts in 256-t1 cells were not reduced by the TNF + CHX treatment at 5 hours (not shown) thus excluding the possibility that loss of the antisense RNA expression, caused by the treatment, may be the reason for lack of protective effects from the necrotic cell death. This further suggests a certain specificity of the protective mechanisms regarding the type of cell killing.

**Table 2:**

| Expression of antisense RNA (from groups 1 and 2) protects from the IFN-γ-induced programmed cell death but not from the TNF-induced necrotic cell death. (A=540 nm) | | | | | | |
|---|---|---|---|---|---|---|
| | | DHFR -t1 | DHFR-t2 | 230-t1 | 255-t1 | 256-t1 |
| 14 days | No treatment | 0.396 | 0.345 | 0.385 | 0.324 | 0.336 |
| | IFN-γ | 0.026 | 0.017 | 0.136 | 0.158 | 0.159 |
| 5 hours | No treatment | N.D. | 0.148 | 0.130 | N.D. | 0.140 |
| | TNF-α + CHX | N.D. | 0.053 | 0.026 | N.D. | 0.022 |
| 20 hours | No treatment | 0.211 | 0.248 | 0.223 | 0.173 | 0.190 |
| | TNF-α + CHX | 0.002 | 0.001 | 0.003 | 0.0015 | 0.002 |

Each treatment was done in quadruplicates and the average values of dye uptake, measured by the OD at 1=540 nm, is presented at the indicated time intervals. The SD was between 2-4%. N.D, not done.

### V. Cloning of DAP-1 cDNA and determination of amino acid sequence.

An HL-60 cDNA library constructed in λgt10 vector was screened with the cDNA insert of pTKO1-230. Two independent clones, λ1 and λ2, almost completely overlapping and carrying cDNA inserts of about 23 Kb were analysed. λ1 cDNA clone encompasses the 5'-untranslated region, short coding region(s) and a relatively long 3'-untranslated region that constitutes more than 60% of the cDNA clone (Fig. 5).

The nucleotide sequence of the cDNA carried by λ1 and its predicted amino acid pattern are presented in Fig. 6. This cDNA is 2232 bp long and contains a potential polyadenylation signal ATTAAA at its 3' end. The open reading frame (ORF) is very short, starting from the initiation codon at nucleotide positions 160-162 and ending at termination codon TGA at positions 466-468. This ORF is preceded by an extremely GC-rich 5'-untranslated region and potentially codes for a protein consisting of 102 amino acids with calculated MW of 11.2 kDa. The amino acid composition predicts a basic protein (isoelectric point = 10), rich in prolines (15%) which displays two blocks of charged residues, one in the middle and the other at the 3' end of the protein. The high proline content may cause some anomalies in the protein's migration on gels. Search for motifs ("Motifs" program; GCG Software Package) indicated that the protein contains two potential sites for casein kinase II phosphorylation at positions 3 and 36, a single potential protein kinase C phosphorylation site at the C-terminus (position 91) and a consensus phosphorylation site of the cdks at position 51. In addition, the protein contains the consensus sequence RGD at position 65-67, a tripeptide that in some proteins plays a role in cell adhesion, and a potential SH3 binding motif, SPSPP, at position 49-53 (Gowburn (1994) Struc. Biol. 1, 489-491). No indications for the presence of signal peptide or transmembranal domain have been found (SAPS prediction; Brendel et al., (1992) PNAS USA, 89:2002-2006). The amino acid sequence showed no significant homology to known proteins.

Fragment #230 was used as a probe on Southern blots containing human genomic DNA, digested with various restriction enzymes that do not cut it. A single band was visualized upon hybridization with DNA cleaved with EcoRI, BamHI, PstI and XbaI, suggesting the existence of a single copy gene (not shown). This new gene was termed DAP-1 (Death Associated Protein-1).

*In vitro* translation assays in reticulocyte lysates confirmed that the predicted ORF codes for the major 15kDa protein translated from the cloned 2.4 Kb transcript. The full length cDNA insert as well as four subclones that span different regions of the molecule (i.e., p6, p5, p8, and p4; see Fig. 5) were transcribed and translated in vitro. Among all the tested subclones, only the 5' 1 Kb portion of the DAP-1 cDNA (p6) directed the in *vitro* synthesis of proteins (Fig. 1C). The major translated product migrated on gels as a 15 kDa protein. Mutation at the ATG codon at position 160-162 (ATG to GGC) completely eliminated the synthesis of the 15 kDa protein, thus confirming the position of the start point of this protein (data not shown). In addition to the 15 kDa protein product, a second protein of 22 kDa was also translated at lower efficiency from λ1 and the p6 cDNAs (Fig . 1C). Its translation was not influenced by the elimination of the ATG codon at position 160 but the protein was shortened to a size of 16 and 18 kDa upon cleavage of the p6 subclone with DraI and BstYI restriction endonucleases, respectively (not shown; for restriction map see Fig. 5). These criteria fit another potential open reading frame, which is detected in the nucleotide sequence in a different phase with respect to the first ORF (Fig. 6). It starts at the ATG codon (positions 287-289) and ends at termination codon TGA (positions 816-818). It has the potential to code for a protein consisting of 176 amino acids with a calculated molecular weight of 19.9 kDa, and has no significant homology to any known proteins.

To analyse the expression of the major DAP-1 protein in cells, rabbit polyclonal antibodies were prepared against the bacterially produced 15kDa protein. The affinity purified antibodies recognized on immunoblots two closely migrating proteins in extracts of HeLa cells; the lower band co-migrated on gels with the bacterially produced 15 kDa DAP-1 protein. The slower inigrating form may represent a post-translationally modified veision of the protein. In the HeLa cell transfectants, 230-t1 and 255-t1, expressing the elevated levels of antisense RNA that develop in the presence of IFN-γ (15 to 1 ratio), the DAP-1 protein levels were reduced by 75% and 78%, respectively, as compared to the DHFR-tranfected cultures (Fig. 1D). The two upper non specific bands (that are not competed with excess of the bacterially produced DAP-1) were not affected by the antisense expression, thus supporting the selectivity of the effect.

### VI. Cloning of DAP-2 and determination of amino acid sequence

As mentioned above, expression studies indicated that the doublestranded cDNA fragment #256 (367 bp in size) hybridized on Northern blots to an endogenous 6.3 Kb mRNA transcript. The same single 6.3Kb mRNA transcript was detected in HeLa (parental and transfectants) and in K562 cells when the full length cDNA (see below) was used as a probe on Northern blots (Fig. 2B). The cDNA insert from pTKO1-256 was therefore used to screen a K562 cDNA library.

Approximately 4x10⁶ pfu were screened with the #256 cDNA insert and 40 positive clones were isolated after two rounds of sequential walking screening. The sequencing was performed on an Applied Bio-systems DNA sequencer 373 A. Sequence uniqueness and relatedness were determined using FASTA (GCG software package) at the nucleotide level and FASTA, BLASTP, and BLOCKS programs at the amino acid level (S. Henikoff and J. G. Henikoff, Nucleic Acids Res. 19, 6565 (1991).

Two clones, λ29 and λ32, were chosen for sequencing (Fig. 7). The resulting composite sequence of both cDNAs consists of 5886 nucleotides and contains a poly A tail that starts at position 5872 and is preceded by two polyadenylation signals AATAAA (Fig. 8). The 3'-untranslated region also contains two ATTTA instability motifs found in the 3'-noncoding portions of short-lived mRNAs (G. Shaw and R. Kamen, Cell 46, 659 (1986)). The mRNA contains a single long open reading frame that starts at position 337, ends at position 4605 and potentially codes for a protein of 1423 amino acids (Fig. 8). The calculated molecular weight of the protein product is about 160 kDa. Affinity purified polyclonal antibodies were raised against the N-terminal 20 amino acid peptide of the protein. These antibodies recognized on immunoblots a 160kDa recombinant protein that was produced in COS-1 cells after transfection with a vector that expressed the entire coding region of the cDNA (Fig. 2D). These antibodies reacted in HeLa cells with an endogenous protein of the same size. In the antisense RNA expressing cells, 256-t1 and 256-t2, the steady state levels of the 160kDa protein were 10 and 5 fold lower than in the DHFR control cells while a non relevant protein, vinculin, displayed similar expression levels in all HeLa cell transfectants (Fig. 2D). Thus, expression of anti-sense RNA from pTKO-1 plasmid #256 in HeLa cells resulted in a significant reduction in the amount of the corresponding protein.

We were able to define several known domains and motifs that are present in this protein. Its extreme N-terminus is composed of a protein kinase domain that spans 255 amino acids from position 13-267. On the basis of its structure, it is likely to be a serine/threonine type of protein kinase having a classical composition of XI subdomains with all conserved motifs present (Fig. 8) (S. K. Hanks and A. M. Quinn, Methods Enzymol. 200, 38 (1991)). This novel kinase was termed DAP-2 or DAP-kinase (Death Associated Proteinkinase).

The kinase domain falls into a family of that of calmodulin-dependent kinases. The homology to known kinase domains that constitute this group, including the myosin light chain kinases, ranges between 34%-49% (Fig. 9A). Three main differences distinguish the kinase domain of DAP-kinase from other members of calmodulin-dependent kinase family: 1) Subdomain II is relatively long and has a stretch of basic amino acids (KKRRTKSSRR); 2) Subdomain III mostly resembles that of the cell cycle dependent kinases (Fig. 9B). Interestingly, the typical sequences of the cell cycle dependent kinases (PSTAIRE, PSSALRE, PCRAIRE, KKIALRE) are located in subdomain III; and 3) Subdomain VII is extremely short and consists of only 7 amino acids.

Right downstream to the kinase domain there is an additional stretch of homology that is present in almost all members of the family of calmodulin-dependent kinases, and was implicated in calmodulin-recognition and binding; B. P. Herring, J. T. Stull, P. J. Gallagher, J. Biol. Chem. 265, 1724 (1990); M. O. Shoemaker *et al*., J.Cell. Biol. 111, 1107 (1990); F.H. Cruzalegui *et al*., Proc. Nath. Acad. Sci. USA 89, 12127 (1992)). Downstream of the calmodulin-recognition domain, an ankyrin repeats domain was identified spanning 265 amino acids from position 365 to 629. It is composed of 8 repeats of 33 amino acids each, not separated by spacets except for a single proline residue that separates three N-terminal repeats from five C-terminal ones (Figs. 8 and 9C). Ankyrin repeats are involved in protein-protein interactions in a variety of proteins (P. Michaely and V. Bennett, Trends in Cell Biology 2, 127 (1992)), but were not described before in the context of serine/threonine kinases. One tyrosine kinase carrying ankyrin repeats has been recently identified in Hydra vulgaris (T.A. Chan *et al*., Oncogene 9, 1253 (1994)). In the DAP-kinase, the 8 ankyrin repeats may mediate the interaction with a putative effector or a regulatory molecule, or influence the substrate selectivity and/or stability of the kinase-substrate interactions.

Immediately downstream to ankyrin repeats there are two subsequent potential P-loop motifs, ALTTDGKT and GHSGSGKT, identified through the consensus sequence, G[A]XXXXGKT[S]. Comparison of DAP-kinase potential P-loop motifs to the corresponding consensus sequences within seven ATP or GTP-binding protein families demonstrates that only the 3' P-loop has some similarity to P-loop consensus of elongation factors, ATP synthase b-subunits and thymidine kinase. Actually, a stretch of 33 amino acids following the eighth ankyrin repeat that encompasses the putative 5' P-loop, may represent a ninth ankyrin repeat that is less conserved than others. DAP-kinase also carries multiple potential sites for post-translational modifications, and has neither transmembranal domain nor signal peptide. The Prosite bank search, using the program Motifs (GCG Software Package) revealed that the DAP-kinase protein contains a consensus sequence for the C-terminal amidation site at position 1376 (this suggests that 47 C-terminal amino acids can be cleaved from the protein body ). It also contains consensus sequences for six N-glycosylation sites, and potential phosphorylation sites for cAMP-dependent kinase (six), casein kinase II (twenty eight) and protein kinase C (twenty).

Altogether, the deduced amino acid sequence of the DAP-kinase suggests that a very unique type of calmodutin-regulated serine/threonine kinase has been rescued. The combination of serine/threonine kinase domain, ankyrin repeats and additional possible ATP/GTP binding sites outside the kinase domain in one protein (Fig. 10) has not been previously described. A size of 160 kDa is rare among serine/threonine kinases and DAP-kinase is actually the largest calmodulin-dependent kinase known to date. The ability of DAP-kinase to bind calmodulin, recently confirmed in yeast two hybrid system (not shown), is consistent with the notion that in many cases programmed cell death is Ca²⁺ dependent (S. Sen, Biol. Rev. Camb. Philos. Soc. 67, 287 (1992); S. Lee, S. Christakos, M. B. Small, Curr. Opin. Cell. Biol. 5, 286 (1993)). Moreover, it has been recently reported that calmodulin antagonists inhibited the glucocorticoid-induced apoptosis (D. R. Dowd, D. P. Mac, B. S. Komm, M. R. Haussler, R. Miesfeld, J. Biol. Chem. 266, 18423 (1991)), and that inhibitors of myosin light chain kinases blocked the TNF-induced apoptotic cell death (S.C. Wright, H. Zheng, J. Zhong, F.M.Torti, J.W. Larrick, J. Cell. Biochem. 53, 222 (1993)).

In order to verify that DAP-2 is truely a kinase, COS cells were transiently transfected with an expression vector (PECE-FLAG) that carries a fragment of the 129 cDNA that encompasses the entire coding region (from the abovementioned start ATG to the first EcoRI site at the 3' end). Cell lysates were immunoprecipitated by anti-FLAG monoclonal antibodies and washed immunoprecipitates were assayed for *in-vitro* autophosphorylation in the presence of calmodulin and Ca²⁺. As shown in Fig. 2C, a single phosphorylated band of 160 kDa appeared upon fractionation of the *in-vitro* reaction products on polyacrylamide gels. This experiment provides the first direct proof that the recombinant protein has intrinsic kinase activity, as suggested by the predicted amino acid structure.

### VII. Expression of DAP-1 and DAP-2 proteins in various cells and tissues

Examination of a variety of cell lines and tissues revealed that these two genes are likely to be ubiquitously expressed. Fig. 10 shows the Northern blot analysis of RNA from different hematopoietic cells probed with the DAP-1 cDNA. The 2.4 Kb mRNA transcript of this gene was detected in granulocytes (HL-60) B lymphoid (Daudi) and macrophage (U937) cells. The expression levels in the hematopoietic cells was lower than in HeLa cells. Fig. 11 shows results of examination of the mRNA expression in human embryonic tissues: brain, spleen (predominantly B cells) and liver (predominantly erythrocytes). Again the single 2.4 Kb mRNA transcript was detected in these tissues by the DAP-1 cDNA probe.

The DAP-2 cDNA probe 2 recognized the 6.3 Kb mRNA encoded by this gene in these different tissues (Fig. 11). The embryonal liver and spleen tissues from Down syndrome seemed in this blot to express higher levels of the DAP-2 gene (compared to the GAPDH levels) while the brain tissue from Down syndrome contained higher levels of DAP-1 mRNA than the corresponding normal brain.

### VIII. Cloning and sequencing of DAP-3, DAP-4 and DAP-5

Clone 259 (DAP-3) was sequenced and used to screen a K562 λgt10 cDNA library as described above for DAP-1 and DAP-2. The sequence of the (almost) full length cDNA of DAP-3 and the deduced amino acid sequence is shown in Fig. 12.

Clones 253 (DAP-4) and 260 (DAP-5) were partially sequenced as described above for DAP-1 and DAP-2, and the results are shown in Figs. 13 and 14, respectively.

### IX. Identification of DAP-7

The initial microscopic observations, performed on the different HeLa cells that had been transfected with the individual rescued pTKO1 clones (described in Table 1), indicated that plasmid pKTO1-229 (group 7) conveyed similar effects to those conferred by the plasmids from group 1. It reduced the susceptibility of the cells to the IFN-γ-induced cell death but not to its cytostatic effects.

The cDNA carried by plasmid pTKO1- 229 was identified upon sequencing as a BamHI-HindIII fragment of human cathepsin D cDNA, which was present in the expression vector in the antisense orientation. The DNA probe, corresponding to fragment #229, hybridized as expected to a single endogenous 2.5 Kb mRNA, both in control and in the transfected HeLa cells. The steady state levels of cathepsin D sense mRNA were not affected by the IFN-γ treatment. In the pTKO1-229 transfected cells the DNA probe also hybridized to the composite antisense RNA. The levels of antisense cathepsin D RNA were stimulated 5-fold in response to IFN-γ due to the presence of an ISRE enhancer element in the pTKO1 expression vector (not shown).

The cathepsin D protein was identified on immunoblots using commercially available polyclonal antibodies (Oncogene Science). It was found that in the control clones. IFN-γ prevented the appearence of the mature 34kDa chain while the 48kDa active single chain precursor was retained at abnormal high levels in these cells. It appears that this single chain precursor is the specific cathepsin form that functions during cell death.

Cathepsin D is an aspartic protease that is found normally in lysosomes where it functions in protein catabolism. Yet, in some pathological situations it has been suggested that this protease can function in the cytosol, and its activity was associated with degenerative brain changes, muscular dystrophy and connective tissue disease pathology (Matus and Green (1987); Biochemistry, 26, 8083-8036). The present invention shows for the first time that the expression of this protease is indispensable for the execution of programmed cell death that is induced by IFN-γ. Thus, cathepsin D joins the growing list of proteases that play a key role in different scenarios of programmed cell death.

The DNA sequence and amino acid sequence of cathepsin D are shown in Fig. 15 (Faust, P.L. *et al*. (1985) PNAS USA 82, 4910-4914).

## Claims

1. A DNA molecule comprising a coding sequence beginning at the nucleic acid triplet at position 160-162 and ending at the triplet at position 466-468 of the sequence depicted in Fig. 6.

2. A DNA molecule encoding the same protein or polypeptide encoded by the DNA sequence of Claim 1.

3. A DNA molecule according to either of claims 1 or 2 in which one or more nucleic acid triplets has been added, deleted or replaced, the protein or polypeptide encoded by the sequence having essentially the same biological activity as that encoded by the DNA molecules of either of claims 1 or 2, respectively, wherein the biological activity is programmed cell death.

4. A fragment of any one of the DNA molecules of Claims 1-3 encoding a protein or polypeptide retaining a biological activity present in the protein or polypeptide encoded by any one of the DNA molecules of either of claims 1 or 2, wherein the biological activity is programmed cell death.

5. A molecule which comprises an antisense sequence complementary in sequence to the mRNA transcribed from the entire or part of the DNA molecule according to Claim 1 and capable of inhibiting the expression of said sequence.

6. A modified DNA sequence of the sequence in Claim 1 in which one or more nucleic acid triplets has been added, deleted or replaced, the protein or polypeptide encoded by the modified sequence having dominant negative effect and being capable of inhibiting the function of the protein or polypeptide encoded by the sequence in Claim 1.

7. A vector comprising a DNA sequence as defined in Claim 1 and sequences required for propagating and replicating the DNA sequence in a host cell.

8. A vector according to Claim 7, being an expression vector comprising also sequences required for transcription of said DNA into a mRNA.

9. A vector according to Claim 8, being an episomal plasmid constructed by using vectors pTKO1, pTKO2 and pTKO3.

10. A protein or polypeptide comprising an amino acid sequence encoded by a DNA molecule as defined in any of Claims 1-4.

11. A protein or polypeptide according to Claim 10, wherein one or more amino acid residues has been chemically modified.

12. A protein according to Claim 10 having the amino acid sequence as depicted in Fig. 6.

13. An antibody directed against a protein according to any one of Claims 10-12 or against an immunogenic portion thereof.

14. A pharmaceutical composition comprising a pharmaceutically acceptable carrier and an active ingredient selected from the group consisting of:
a. a protein or polypeptide according to any one of claims 10-12;
b. an expression vector according to either of Claims 8 or 9;
c. an antibody according to claim 13; and
d. an inhibitor or antagonist of the protein of (a).

15. A pharmaceutical composition according to claim 14, for inducing programmed cell death, wherein said active ingredient is selected from the group consisting of:
a. a protein according to any one of Claims 10-12; and
b. an expression vector according to claim 8 in which the DNA molecule is that of claims 1-4.

16. A pharmaceutical composition according to claim 15 for inducing cytokine-induced programmed cell death.

17. A pharmaceutical composition according to claim 16, further comprising a cytokine.

18. A pharmaceutical composition for use in inhibiting cell destruction caused by programmed cell death, comprising a pharmaceutically acceptable carrier and an active agent being:
a. an antibody of claim 13;
b. an inhibitor or antagonist of the protein of Claims 10-12; and
c. an expression vector according to claim 8 in which the DNA molecule is that of either of claims 5 or 6.

19. A method for detecting a mutation in a gene encoding a product involved in programmed cell death comprising probing genomic DNA or cDNA from the individual with one or more DNA probes comprising a complete or partial sequence of the DNA molecules as defined in claims 1-4.

20. A method for the screening of individuals for a predisposition to cancer, comprising:
(a) obtaining a sample of either genomic DNA from cells of the individual or cDNA produced from mRNA of said cells;
(b) adding one or more DNA probes, each of said probes comprising a DNA sequence as defined in claims 1-4
(c) providing conditions for hybridization between the DNA probe or probes and the DNA of said samples;
(d) determining on the basis of the hybridization whether the DNA sequence as defined in claim 1 is absent, whether there is a match between the sequence of the DNA probe or probes and a sequence in the DNA of said sample or whether there is a mismatch, a mismatch or absence indicating a deletion or a mutation in the genomic DNA and a predisposition to cancer of the tested individual.

21. A method for screening of individuals for a predisposition to cancer by the use of antibodies which are able to distinguish between a functional protein of Claim 10 and a non-functional mutated protein.

22. A method of treatment of a disease or a disorder associated with uncontrolled, pathological cell growth, comprising administering to a diseased individual a therapeutically effective amount of an active ingredient selected from the group consisting of:
a. a protein according to any one of Claims 10-12; and
b. an expression vector according to claim 8 in which the DNA molecule is that of claims 1-4.

23. A method of treatment of a disease according to claim 22, wherein said active ingredient is administered in conjunction with a cytokine.

24. A method of treatment of a disease or disorder associated with cytokine-induced programmed cell death, comprising administering a therapeutically effective amount of an active ingredient capable of inhibiting physiological cell death, said active ingredient being a member selected from the group consisting of:
a. an antibody of claim 13;
b. an inhibitor or antagonist of the protein of Claims 10-12; and
c. an expression vector according to either of claims 8 or 9 in which the DNA molecule is that of either of claims 5 or 6.

25. A DNA probe comprising a complete or partial sequence of the DNA molecule as defined in claims 1-4, or having a sequence which is an antisense to the complete or partial sequence of the DNA molecule as defined in claims 1-4, and is capable of hybridizing to said DNA molecule.


SEQUENCE LISTING

(1) GENERAL INFORMATION:

(i) APPLICANT:
(A) NAME: YEDA RESEARCH AND DEVELOPMENT COMPANY LTD.
(B) STREET: The Weizmann Institute of Science - P.O. Box
95
(C) CITY: REHOVOT
(E) COUNTRY: ISRAEL
(F) POSTAL CODE (ZIP): 76100

(ii) TITLE OF INVENTION: TUMOR SUPPRESSOR GENES, PROTEINS ENCODED
THEREBY AND USE OF SAID GENES AND PROTEINS

(iii) NUMBER OF SEQUENCES: 26

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: 05005471.7
(vi) PRIOR APPLICATION DATA:
(A) APPLICATION NUMBER: EP 95900994.5
(B) FILING DATE: 12-OCT-1994
(vi) PRIOR APPLICATION DATA:
(A) APPLICATION NUMBER: PCT/US94/11598
(B) FILING DATE: 12-OCT-1994

(vi) PRIOR APPLICATION DATA:
(A) APPLICATION NUMBER: IL 107250
(B) FILING DATE: 12-OCT-1993

(2) INFORMATION FOR SEQ ID NO: 1:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 1:

Lys Lys Arg Arg Thr Lys Ser Ser Arg Arg
1               5                   10

(2) INFORMATION FOR SEQ ID NO: 2:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 2:

Pro Ser Thr Ala Ile Arg Glu
1               5

(2) INFORMATION FOR SEQ ID NO: 3:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 3:

Pro Ser Ser Ala Leu Arg Glu
1               5

(2) INFORMATION FOR SEQ ID NO: 4:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 4:

Pro Cys Thr Ala Ile Arg Glu
1               5

(2) INFORMATION FOR SEQ ID NO: 5:

(i) SEQUENCE CHARACTERISTICS:
(A) LENGTH: 7 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 5:

Lys Lys Ile Ala Leu Arg Glu

1               5

(2) INFORMATION FOR SEQ ID NO: 6:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 6:

Ala Leu Thr Thr Asp Gly Lys Thr
1               5

(2) INFORMATION FOR SEQ ID NO: 7:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 7:

Gly His Ser Gly Ser Gly Lys Thr
1               5

(2) INFORMATION FOR SEQ ID NO: 8:

(i) SEQUENCE CHARACTERISTICS:
(A) LENGTH: 8 amino acids
(B) TYPE: amino acid
(C) STRANDEDNESS: single
(D) TOPOLOGY: linear

(ii) MOLECULE TYPE: protein

(ix) FEATURE:
(A) NAME/KEY: Protein
(B) LOCATION:1..2
(D) OTHER INFORMATION:/note= "G at this location can be
A"

(ix) FEATURE:
(A) NAME/KEY: Protein
(B) LOCATION:7..8
(D) OTHER INFORMATION:/note= "T at this location can be
S"

(xi) SEQUENCE DESCRIPTION: SEQ ID NO: 8:

Gly Xaa Xaa Xaa Xaa Gly Lys Thr
1               5

(2) INFORMATION FOR SEQ ID NO: 9:

(i) SEQUENCE CHARACTERISTICS:
(A) LENGTH: 2232 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: CDS
(B) LOCATION:160..468
(D) OTHER INFORMATION:/note= "prevention of IFN-2
promoted cell death"

(xi) SEQUENCE DESCRIPTION: SEQ ID NO: 9:

CGTGGCACTC ACCCGGCTCG CGCGGCCCCG GCCGCCCACG CCGCGCGTCG TTCTCCCGCC      60

CGCTCGCTCC CCGGCGCTCA CACCTGAGCT CACTCGCGCA CGCCCGCCCG GCCCGAGAAC     120

CGCGCCGCCG CCTCGGCCCC GCGGAAGCCC CGCCGCGCC ATG TCT TCG CCT CCC     174
                                          Met Ser Ser Pro Pro
                                            1               5

GAA GGG AAA CTA GAG ACT AAA GCT GGA CAC CCG CCC GCC GTG AAA GCT     222
Glu Gly Lys Leu Glu Thr Lys Ala Gly His Pro Pro Ala Val Lys Ala
                 10                  15                  20

GGT GGA ATG CGA ATT GTG CAG AAA CAC CCA CAT ACA GGA GAC ACC AAA     270
Gly Gly Met Arg Ile Val Gln Lys His Pro His Thr Gly Asp Thr Lys
             25                  30                  35

GAA GAG AAA GAC AAG GAT GAC CAG GAA TGG GAA AGC CCC AGT CCA CCT     318
Glu Glu Lys Asp Lys Asp Asp Gln Glu Trp Glu Ser Pro Ser Pro Pro
         40                  45                  50

AAA CCC ACT GTG TTC ATC TCT GGG GTC ATC GCC CGG GGT GAC AAA GAT     366
Lys Pro Thr Val Phe Ile Ser Gly Val Ile Ala Arg Gly Asp Lys Asp
     55                  60                  65

TTC CCC CCG GCG GCT GCG CAG GTG GCT CAC CAG AAG CCG CAT GCC TCC     414
Phe Pro Pro Ala Ala Ala Gln Val Ala His Gln Lys Pro His Ala Ser
 70                  75                  80                  85

ATG GAC AAG CAT CCT TCC CCA AGA ACC CAG CAC ATC CAG CAG CCA CGC     462
Met Asp Lys His Pro Ser Pro Arg Thr Gln His Ile Gln Gln Pro Arg
                 90                  95                 100

AAG TGA GCCTGGAGTC CACCAGCCTG CCCCATGGCC CCGGCTCTGC TGCACTTGGT     518
Lys  *

ATTTCCCTGA CAGAGAGAAC CAGCAGTTTC GCCCAAATCC TACTCTGCTG GGAAATCTAA     578

GGCAAAACCA AGTGCTCTGT CCTTTGCCTT ACATTTCCAT ATTTAAAACT AGAAACAGCT     638

TCAGCCCAAA CCTTGTTTAT GGGGAGTCTG GTTGGATGTC ATTTGAGGAT CATTGTGCCC 698

CTAGAGGTGC CATTAGCAGA ATTTGCCAAG ATCCGAGAAA AATTTTAGCT TTAGTTCTAT 758

TTCAGCAGTC ACCTGACGTC CTTGTCTATG GTCTTAAAAA CAAGAAGGCA CACATTTGAG 818

AAGATGAGAT TAAGGTTAGG AGAAAACCTC AGTCATTGCA TGCTTTTTAG TATGGGCCAA 878

TAAAATCTCA ACACCTGTGG GAGAGTAAGA ACTAAGGGAA TGAGTTTGGG CGCCCCCTCA 938

TAAAGGACCT TAGAGGCAGG GAACAGCAAT GCCAAATTTC CCTCTCTCGT GAGATGGGGG 998

ATCCTGTGCA GGCTGATGAG GCACCCATGA GAAAAGCCGA AAAAGCATGC ATCTTAGAAA 1058

TAGCCCCTCA ATTCCAGGAG TCAACATGCC AAAGAATGAG GCTGGAGACA GGTAGCTCCG 1118

AGGGAGGACT TCTGGCATGA GATCTCGGCA CGGCAAGCCC AGCATCGCCT CAGCCCAGAC 1178

AGGCTCCACC AGGAGATCAA GCAAGGGCTG CCTTTCAGGA GTCACCTCCT GAGCCACTTC 1238

AGAGTTCTGG AAGTGACCAC GGACCAGGGT GGAGGAATAG ACTTCTAGTT CATTCTGGGA 1298

CACTTGAGCC AGAGAGTTGA AAGCTTGGAA AGACCAGATA AGAAACCTGC CCTTTGTCTC 1358

CCTAGGGACA TGAGACACCA CATTCCATTT GTGCTAGAAA AACCTATCCA CTGATGAGTC 1418

TAACTGTTCC AAACGCCTCC CACCTGGTGT GCACAGCTGC CTGGGTCCAT TGTCACTTGG 1478

GTGCATCAGG TTGTCCTCCG ATTTTTAGAT GAGTTTCCTG TCTAGAGATG TCCTAGTCTG 1538

CTCACTGGCT GGTGGCAGTA GGGTACCCTG CGTCCTCGAA AAGCCAGAGG GTTCACCTAG 1598

TCAGACGAAA CTCCAGAACA GTGCTTGTGG AGGGCCTGAC TGTCCTGCTC ACCCACAGCC 1658

GATCTGCTGC AGGTCAGCAA CTGTGTCGTG AGCAGCTGCC AACCACCAGC CTTTCTGGTG 1718

CTGTTCTCCA GTTCACGTCT GCCAGCTGGT GAGGGCAGAG GCAGACCTGG TCAGACCCAG 1778

CGCCCCTCCT CCCTGAGGGA GCATGGCACA GCCTCACACT TGAAAGACGG TGTTTGGTTT 1838

CCCATCTAAT CAACTTAAGG GAAGCCGGCA TGTACCCTTC AAGGCCCTGT CACCACCTAT 1898

TTTCCTGATC AGTTGGTATA AACTGAGGGT GGCTTTTAGA GACCCAGACT TGGTTGGCAG 1958

CGCTGCCATG GAACACCCCA GCAAGCACCT CCCAGCCTGC CTTTCGGAGC AGCACCCAGG 2018

AGGGGATGCC GCGCTCCAGC AACACCAGGT CAGGCCTGTG CAGACCCCTG CCCTGCCGCT 2078

GCAGAAATCC AGAAGCATCC TTAATGCTTC TCAGTCTTCA GCCAGAGGGA GGGCTGTTAT 2138

TTCCAGAGGT GCGCTTTTTA TGTACTTTTA GCTAGATGTG GCATGCATCT GTGAGCTTTA 2198

GATCATTAAA TCCAAAATGT TTGCCTAAAT GAGG 2232

(2) INFORMATION FOR SEQ ID NO: 10:

(i) SEQUENCE CHARACTERISTICS:
(A) LENGTH: 103 amino acids
(B) TYPE: amino acid
(D) TOPOLOGY: linear

(ii) MOLECULE TYPE: protein
(xi) SEQUENCE DESCRIPTION: SEQ ID NO: 10:

Met Ser Ser Pro Pro Glu Gly Lys Leu Glu Thr Lys Ala Gly His Pro
1               5                   10                  15

Pro Ala Val Lys Ala Gly Gly Met Arg Ile Val Gln Lys His Pro His
            20                  25                  30

Thr Gly Asp Thr Lys Glu Glu Lys Asp Lys Asp Asp Gln Glu Trp Glu
        35                  40                  45

Ser Pro Ser Pro Pro Lys Pro Thr Val Phe Ile Ser Gly Val Ile Ala
    50                  55                  60

Arg Gly Asp Lys Asp Phe Pro Pro Ala Ala Ala Gln Val Ala His Gln
65                  70                  75                  80

Lys Pro His Ala Ser Met Asp Lys His Pro Ser Pro Arg Thr Gln His
                85                  90                  95

Ile Gln Gln Pro Arg Lys  *
            100

(2) INFORMATION FOR SEQ ID NO: 11:

(i) SEQUENCE CHARACTERISTICS:
(A) LENGTH: 5886 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: CDS
(B) LOCATION:337..4605
(D) OTHER INFORMATION:/note= "prevention of IFN-2
promoted cell death"

(xi) SEQUENCE DESCRIPTION: SEQ ID NO: 11:

CGGAGGACAG CCGGACCGAG CCAACGCCGG GGACTTTGTT CCCTCCACGG AGGGGACTCG      60

GCAACTCGCA GCGGCAGGGT CTGGGGCCGG CGCCTGGGAG GGATCTGCGC CCCCCACTCA     120

CTCCCTAGCT GTGTTCCCGC CGCCGCCCCG GCTAGTCTCC GGCGCTGGCG CCTATGGTCG     180

GCCTCCGACA GCGCTCCGGA GGGACCGGGG GAGCTCCCAG GCGCCCGGGA CTGGAGACTG     240

ATGCATGAGG GGCCTACGGA GGCGCAGGAG CGGTGGTGAT GGTCTGGGAA GCGGAGCTGA     300

AGTCCCCTGG GCTTTGGTGA GGCGTGACAG TTTATC ATG ACC GTG TTC AGG CAG     354
                                        Met Thr Val Phe Arg Gln
                                                105

GAA AAC GTG GAT GAT TAC TAC GAC ACC GGC GAG GAA CTT GGC AGT GGA     402
Glu Asn Val Asp Asp Tyr Tyr Asp Thr Gly Glu Glu Leu Gly Ser Gly
110                 115                 120                 125

CAG TTT GCG GTT GTG AAG AAA TGC CGT GAG AAA AGT ACC GGC CTC CAG     450
Gln Phe Ala Val Val Lys Lys Cys Arg Glu Lys Ser Thr Gly Leu Gln
                130                 135                 140

TAT CCC GCC AAA TTC ATC AAG AAA AGG AGG ACT AAG TCC AGC CGG CGG     498
Tyr Pro Ala Lys Phe Ile Lys Lys Arg Arg Thr Lys Ser Ser Arg Arg
            145                 150                 155

GGT GTG AGC CGC GAG GAC ATC GAG CGG GAG GTC AGC ATC CTG AAG GAG     546
Gly Val Ser Arg Glu Asp Ile Glu Arg Glu Val Ser Ile Leu Lys Glu
        160                 165                 170

ATC CAG CAC CCC AAT GTC ATC ACC CTG CAC GAG GTC TAT GAG AAC AAG     594
Ile Gln His Pro Asn Val Ile Thr Leu His Glu Val Tyr Glu Asn Lys
    175                 180                 185

ACG GAC GTC ATC CTG ATC TTG GAA CTC GTT GCA GGT GGC GAG CTG TTT     642
Thr Asp Val Ile Leu Ile Leu Glu Leu Val Ala Gly Gly Glu Leu Phe
190                 195                 200                 205

GAC TTC TTA GCT GAA AAG GAA TCT TTA ACT GAA GAG GAA GCA ACT GAA     690
Asp Phe Leu Ala Glu Lys Glu Ser Leu Thr Glu Glu Glu Ala Thr Glu
                210                 215                 220

TTT CTC AAA CAA ATT CTT AAT GGT GTT TAC TAC CTG CAC TCC CTT CAA     738
Phe Leu Lys Gln Ile Leu Asn Gly Val Tyr Tyr Leu His Ser Leu Gln
            225                 230                 235

ATC GCC CAC TTT GAT CTT AAG CCT GAG AAC ATA ATG CTT TTG GAT AGA     786
Ile Ala His Phe Asp Leu Lys Pro Glu Asn Ile Met Leu Leu Asp Arg
        240                 245                 250

AAT GTC CCC AAA CCT CGG ATC AAG ATC ATT GAC TTT GGA AAT GAA TTT     834
Asn Val Pro Lys Pro Arg Ile Lys Ile Ile Asp Phe Gly Asn Glu Phe
    255                 260                 265

AAA AAC ATA TTT GGG ACT CCA GAG TTT GTC GCT CCT GAG ATA GTC AAC     882
Lys Asn Ile Phe Gly Thr Pro Glu Phe Val Ala Pro Glu Ile Val Asn
270                 275                 280                 285

TAT GAA CCT CTT GGT CTT GAG GCA GAT ATG TGG AGT ATC GGG GTA ATA     930
Tyr Glu Pro Leu Gly Leu Glu Ala Asp Met Trp Ser Ile Gly Val Ile
                290                 295                 300

ACC TAT ATC CTC CTA AGT GGG GCC TCC CCA TTT CTT GGA GAC ACT AAG     978
Thr Tyr Ile Leu Leu Ser Gly Ala Ser Pro Phe Leu Gly Asp Thr Lys
            305                 310                 315

CAA GAA ACG TTA GCA AAT GTA TCC GCT GTC AAC TAC GAA TTT GAG GAT     1026
Gln Glu Thr Leu Ala Asn Val Ser Ala Val Asn Tyr Glu Phe Glu Asp
        320                 325                 330

GAA TAC TTC AGT AAT ACC AGT GCC CTA GCC AAA GAT TTC ATA AGA AGA     1074
Glu Tyr Phe Ser Asn Thr Ser Ala Leu Ala Lys Asp Phe Ile Arg Arg
    335                 340                 345

CTT CTG GTC AAG GAT CCA AAG AAG AGA ATG ACA ATT CAA GAT AGT TTG 1122
Leu Leu Val Lys Asp Pro Lys Lys Arg Met Thr Ile Gln Asp Ser Leu
350 355 360 365

CAG CAT CCC TGG ATC AAG CCT AAA GAT ACA CAA CAG GCA CTT AGT AGA 1170
Gln His Pro Trp Ile Lys Pro Lys Asp Thr Gln Gln Ala Leu Ser Arg
370 375 380

AAA GCA TCA GCA GTA AAC ATG GAG AAA TTC AAG AAG TTT GCA GCC CGG 1218
Lys Ala Ser Ala Val Asn Met Glu Lys Phe Lys Lys Phe Ala Ala Arg
385 390 395

AAA AAA TGG AAA CAA TCC GTT CGC TTG ATA TCA CTG TGC CAA AGA TTA 1266
Lys Lys Trp Lys Gln Ser Val Arg Leu Ile Ser Leu Cys Gln Arg Leu
400 405 410

TCC AGG TCA TTC CTG TCC AGA AGT AAC ATG AGT GTT GCC AGA AGC GAT 1314
Ser Arg Ser Phe Leu Ser Arg Ser Asn Met Ser Val Ala Arg Ser Asp
415 420 425

GAT ACT CTG GAT GAG GAA GAC TCC TTT GTG ATG AAA GCC ATC ATC CAT 1362
Asp Thr Leu Asp Glu Glu Asp Ser Phe Val Met Lys Ala Ile Ile His
430 435 440 445

GCC ATC AAC GAT GAC AAT GTC CCA GGC CTG CAG CAC CTT CTG GGC TCA 1410
Ala Ile Asn Asp Asp Asn Val Pro Gly Leu Gln His Leu Leu Gly Ser
450 455 460

TTA TCC AAC TAT GAT GTT AAC CAA CCC AAC AAG CAC GGG ACA CCT CCA 1458
Leu Ser Asn Tyr Asp Val Asn Gln Pro Asn Lys His Gly Thr Pro Pro
465 470 475

TTA CTC ATT GCT GCT GGC TGT GGG AAT ATT CAA ATA CTA CAG TTG CTC 1506
Leu Leu Ile Ala Ala Gly Cys Gly Asn Ile Gln Ile Leu Gln Leu Leu
480 485 490

ATT AAA AGA GGC TCG AGA ATC GAT GTC CAG GAT AAG GGC GGG TCC AAT 1554
Ile Lys Arg Gly Ser Arg Ile Asp Val Gln Asp Lys Gly Gly Ser Asn
495 500 505

GCC GTC TAC TGG GCT GCT CGG CAT GGC CAC GTC GAT ACC TTG AAA TTT 1602
Ala Val Tyr Trp Ala Ala Arg His Gly His Val Asp Thr Leu Lys Phe
510 515 520 525

CTC AGT GAG AAC AAA TGC CCT TTG GAT GTG AAA GAC AAG TCT GGA GAG 1650
Leu Ser Glu Asn Lys Cys Pro Leu Asp Val Lys Asp Lys Ser Gly Glu
530 535 540

ATG GCC CTC CAC GTG GCA GCT CGC TAT GGC CAT GCT GAC GTG GCT CAA 1698
Met Ala Leu His Val Ala Ala Arg Tyr Gly His Ala Asp Val Ala Gln
545 550 555

GTT ACT TGT GCA GCT TCG GCT CAA ATC CCA ATA TCC AGG ACA AAG GAA 1746
Val Thr Cys Ala Ala Ser Ala Gln Ile Pro Ile Ser Arg Thr Lys Glu
560 565 570

GAA GAA ACC CCC CTG CAC TGT GCT GCT TGG CAC GGC TAT TAC TCT GTG 1794
Glu Glu Thr Pro Leu His Cys Ala Ala Trp His Gly Tyr Tyr Ser Val
575 580 585

GCC AAA GCC CTT TGT GAA GCC GGC TGT AAC GTG AAC ATC AAG AAC CGA 1842
Ala Lys Ala Leu Cys Glu Ala Gly Cys Asn Val Asn Ile Lys Asn Arg
590 595 600 605

GAA GGA GAG ACG CCC CTC CTG ACA GCC TCT GCC AGG GGC TAC CAC GAC 1890
Glu Gly Glu Thr Pro Leu Leu Thr Ala Ser Ala Arg Gly Tyr His Asp
610 615 620

ATC GTG GAG TGT CTG GCC GAA CAT GGA GCC GAC CTT AAT GCT TGC GAC 1938
Ile Val Glu Cys Leu Ala Glu His Gly Ala Asp Leu Asn Ala Cys Asp
625 630 635

AAG GAC GGA CAC ATT GCC CTT CAT CTG GCT GTA AGA CGG TGT CAG ATG 1986
Lys Asp Gly His Ile Ala Leu His Leu Ala Val Arg Arg Cys Gln Met
640 645 650

GAG GTA ATC AAG ACT CTC CTC AGC CAA GGG TGT TTC GTC GAT TAT CAA 2034
Glu Val Ile Lys Thr Leu Leu Ser Gln Gly Cys Phe Val Asp Tyr Gln
655 660 665

GAC AGG CAC GGC AAT ACT CCC CTC CAT GTG GCA TGT AAA GAT GGC AAC 2082
Asp Arg His Gly Asn Thr Pro Leu His Val Ala Cys Lys Asp Gly Asn
670 675 680 685

ATG CCT ATC GTG GTG GCC CTC TGT GAA GCA AAC TGC AAT TTG GAC ATC 2130
Met Pro Ile Val Val Ala Leu Cys Glu Ala Asn Cys Asn Leu Asp Ile
690 695 700

TCC AAC AAG TAT GGG CGA ACG CCT CTG CAC CTT GCG GCC AAC AAC GGA 2178
Ser Asn Lys Tyr Gly Arg Thr Pro Leu His Leu Ala Ala Asn Asn Gly
705 710 715

ATC CTA GAC GTG GTC CGG TAT CTC TGT CTG ATG GGA GCC AGC GTT GAG 2226
Ile Leu Asp Val Val Arg Tyr Leu Cys Leu Met Gly Ala Ser Val Glu
720 725 730

GCG CTG ACC ACG GAC GGA AAG ACG GCA GAA GAT CTT GCT AGA TCG GAA 2274
Ala Leu Thr Thr Asp Gly Lys Thr Ala Glu Asp Leu Ala Arg Ser Glu
735 740 745

CAG CAC GAG CAC GTA GCA GGT CTC CTT GCA AGA CTT CGA AAG GAT ACG 2322
Gln His Glu His Val Ala Gly Leu Leu Ala Arg Leu Arg Lys Asp Thr
750 755 760 765

CAC CGA GGA CTC TTC ATC CAG CAG CTC CGA CCC ACA CAG AAC CTG CAG 2370
His Arg Gly Leu Phe Ile Gln Gln Leu Arg Pro Thr Gln Asn Leu Gln
770 775 780

CCA AGA ATT AAG CTC AAG CTG TTT GGC CAC TCG GGA TCC GGG AAA ACC 2418
Pro Arg Ile Lys Leu Lys Leu Phe Gly His Ser Gly Ser Gly Lys Thr
785 790 795

ACC CTT GTA GAA TCT CTC AAG TGT GGG CTG CTG AGG AGC TTT TTC AGA 2466
Thr Leu Val Glu Ser Leu Lys Cys Gly Leu Leu Arg Ser Phe Phe Arg
800 805 810

AGG CGT CGG CCC AGA CTG TCT TCC ACC AAC TCC AGC AGG TTC CCA CCT 2514
Arg Arg Arg Pro Arg Leu Ser Ser Thr Asn Ser Ser Arg Phe Pro Pro
815 820 825

TCA CCC CTG GCT TCT AAG CCC ACA GTC TCA GTG AGC ATC AAC AAC CTG 2562
Ser Pro Leu Ala Ser Lys Pro Thr Val Ser Val Ser Ile Asn Asn Leu
830 835 840 845

TAC CCA GGC TGC GAG AAC GTG AGT GTG AGG AGC CGC AGC ATG ATG TTC 2610
Tyr Pro Gly Cys Glu Asn Val Ser Val Arg Ser Arg Ser Met Met Phe
850 855 860

GAG CCG GGT CTT ACC AAA GGG ATG CTG GAG GTG TTT GTG GCC CCG ACC 2658
Glu Pro Gly Leu Thr Lys Gly Met Leu Glu Val Phe Val Ala Pro Thr
865 870 875

CAC CAC CCG CAC TGC TCG GCC GAT GAC CAG TCC ACC AAG GCC ATC GAC 2706
His His Pro His Cys Ser Ala Asp Asp Gln Ser Thr Lys Ala Ile Asp
880 885 890

ATC CAG AAC GCT TAT TTG AAT GGA GTT GGC GAT TTC AGC GTG TGG GAG 2754
Ile Gln Asn Ala Tyr Leu Asn Gly Val Gly Asp Phe Ser Val Trp Glu
895 900 905

TTC TCT GGA AAT CCT GTG TAT TTC TGC TGT TAT GAC TAT TTT GCT GCA 2802
Phe Ser Gly Asn Pro Val Tyr Phe Cys Cys Tyr Asp Tyr Phe Ala Ala
910 915 920 925

AAT GAT CCC ACG TCA ATC CAT GTT GTT GTC TTT AGT CTA GAA GAG CCC 2850
Asn Asp Pro Thr Ser Ile His Val Val Val Phe Ser Leu Glu Glu Pro
930 935 940

TAT GAG ATC CAG CTG AAC CCA GTG ATT TTC TGG CTC AGT TTC CTG AAG 2898
Tyr Glu Ile Gln Leu Asn Pro Val Ile Phe Trp Leu Ser Phe Leu Lys
945 950 955

TCC CTT GTC CCA GTT GAA GAA CCC ATA GCC TTC GGT GGC AAG CTG AAG 2946
Ser Leu Val Pro Val Glu Glu Pro Ile Ala Phe Gly Gly Lys Leu Lys
960 965 970

AAC CCA CTC CAA GTT GTC CTG GTG GCC ACC CAC GCT GAC ATC ATG AAT 2994
Asn Pro Leu Gln Val Val Leu Val Ala Thr His Ala Asp Ile Met Asn
975 980 985

GTT CCT CGA CCG GCT GGA GGC GAG TTT GGA TAT GAC AAA GAC ACA TCG 3042
Val Pro Arg Pro Ala Gly Gly Glu Phe Gly Tyr Asp Lys Asp Thr Ser
990 995 1000 1005

TTG CTG AAA GAG ATT AGG AAC AGG TTT GGA AAT GAT CTT CAC ATT TCA 3090
Leu Leu Lys Glu Ile Arg Asn Arg Phe Gly Asn Asp Leu His Ile Ser
1010 1015 1020

AAT AAG CTG TTT GTT CTG GAT GCT GGG GCT TCT GGG TCA AAG GAC ATG 3138
Asn Lys Leu Phe Val Leu Asp Ala Gly Ala Ser Gly Ser Lys Asp Met
1025 1030 1035

AAG GTA CTT CGA AAT CAT CTG CAA GAA ATA CGA AGC CAG ATT GTT TCG 3186
Lys Val Leu Arg Asn His Leu Gln Glu Ile Arg Ser Gln Ile Val Ser
1040 1045 1050

GTC TGT CCT CCC ATG ACT CAC CTG TGT GAG AAA ATC ATC TCC ACG CTG 3234
Val Cys Pro Pro Met Thr His Leu Cys Glu Lys Ile Ile Ser Thr Leu
1055 1060 1065

CCT TCC TGG AGG AAG CTC AAT GGA CCC AAC CAG CTG ATG TCG CTG CAG 3282
Pro Ser Trp Arg Lys Leu Asn Gly Pro Asn Gln Leu Met Ser Leu Gln
1070 1075 1080 1085

CAG TTT GTG TAC GAC GTG CAG GAC CAG CTG AAC CCC CTG GCC AGC GAG 3330
Gln Phe Val Tyr Asp Val Gln Asp Gln Leu Asn Pro Leu Ala Ser Glu
1090 1095 1100

GAG GAC CTC AGG CGC ATT GCT CAG CAG CTC CAC AGC ACA GGC GAG ATC 3378
Glu Asp Leu Arg Arg Ile Ala Gln Gln Leu His Ser Thr Gly Glu Ile
1105 1110 1115

AAC ATC ATG CAA AGT GAA ACA GTT CAG GAC GTG CTG CTC CTG GAC CCC 3426
Asn Ile Met Gln Ser Glu Thr Val Gln Asp Val Leu Leu Leu Asp Pro
1120 1125 1130

CGC TGG CTC TGC ACA AAC GTC CTG GGG AAG TTG CTG TCC GTG GAG ACC 3474
Arg Trp Leu Cys Thr Asn Val Leu Gly Lys Leu Leu Ser Val Glu Thr
1135 1140 1145

CCA CGG GCG CTG CAC CAC TAC CGG GGC CGC TAC ACC GTG GAG GAC ATC 3522
Pro Arg Ala Leu His His Tyr Arg Gly Arg Tyr Thr Val Glu Asp Ile
1150 1155 1160 1165

CAG CGC CTG GTG CCC GAC AGC GAC GTG GAG GAG CTG CTG CAG ATC CTC 3570
Gln Arg Leu Val Pro Asp Ser Asp Val Glu Glu Leu Leu Gln Ile Leu
1170 1175 1180

GAT GCC ATG GAC ATC TGC GCC CGG GAC CTG AGC AGC GGG ACC ATG GTG 3618
Asp Ala Met Asp Ile Cys Ala Arg Asp Leu Ser Ser Gly Thr Met Val
1185 1190 1195

GAC GTC CCA GCC CTG ATC AAG ACA GAC AAC CTG CAC CGC TCC TGG GCT 3666
Asp Val Pro Ala Leu Ile Lys Thr Asp Asn Leu His Arg Ser Trp Ala
1200 1205 1210

GAT GAG GAG GAC GAG GTG ATG GTG TAT GGT GGC GTG CGC ATC GTG CCC 3714
Asp Glu Glu Asp Glu Val Met Val Tyr Gly Gly Val Arg Ile Val Pro
1215 1220 1225

GTG GAA CAC CTC ACC CCC TTC CCA TGT GGC ATC TTT CAC AAG GTC CAG 3762
Val Glu His Leu Thr Pro Phe Pro Cys Gly Ile Phe His Lys Val Gln
1230 1235 1240 1245

GTG AAC CTG TGC CGG TGG ATC CAC CAG CAA AGC ACA GAG GGC GAC GCG 3810
Val Asn Leu Cys Arg Trp Ile His Gln Gln Ser Thr Glu Gly Asp Ala
1250 1255 1260

GAC ATC CGC CTG TGG GTG AAT GGC TGC AAG CTG GCC AAC CGT GGG GCC 3858
Asp Ile Arg Leu Trp Val Asn Gly Cys Lys Leu Ala Asn Arg Gly Ala
1265 1270 1275

GAG CTG CTG GTG CTG CTG GTC AAC CAC GGC CAG GGC ATT GAG GTC CAG 3906
Glu Leu Leu Val Leu Leu Val Asn His Gly Gln Gly Ile Glu Val Gln
1280 1285 1290

GTC CGT GGC CTG GAG ACG GAG AAG ATC AAG TGC TGC CTG CTG CTG GAC 3954
Val Arg Gly Leu Glu Thr Glu Lys Ile Lys Cys Cys Leu Leu Leu Asp
1295 1300 1305

TCG GTG TGC AGC ACC ATT GAG AAC GTC ATG GCC ACC ACG CTG CCA GGG 4002
Ser Val Cys Ser Thr Ile Glu Asn Val Met Ala Thr Thr Leu Pro Gly
1310 1315 1320 1325

CTC CTG ACC GTG AAG CAT TAC CTG AGC CCC CAG CAG CTG CGG GAG CAC 4050
Leu Leu Thr Val Lys His Tyr Leu Ser Pro Gln Gln Leu Arg Glu His
1330 1335 1340

CAT GAG CCC GTC ATG ATC TAC CAG CCA CGG GAC TTC TTC CGG GCA CAG 4098
His Glu Pro Val Met Ile Tyr Gln Pro Arg Asp Phe Phe Arg Ala Gln
1345 1350 1355

ACT CTG AAG GAA ACC TCA CTG ACC AAC ACC ATG GGG GGG TAC AAG GAA 4146
Thr Leu Lys Glu Thr Ser Leu Thr Asn Thr Met Gly Gly Tyr Lys Glu
1360 1365 1370

AGC TTC AGC AGC ATC ATG TGC TTC GGG TGT CAC GAC GTC TAC TCA CAG 4194
Ser Phe Ser Ser Ile Met Cys Phe Gly Cys His Asp Val Tyr Ser Gln
1375 1380 1385

GCC AGC CTC GGC ATG GAC ATC CAT GCA TCA GAC CTG AAC CTC CTC ACT 4242
Ala Ser Leu Gly Met Asp Ile His Ala Ser Asp Leu Asn Leu Leu Thr
1390 1395 1400 1405

CGG AGG AAA CTG AGT CGC CTG CTG GAC CCG CCC GAC CCC CTG GGG AAG 4290
Arg Arg Lys Leu Ser Arg Leu Leu Asp Pro Pro Asp Pro Leu Gly Lys
1410 1415 1420

GAC TGG TGC CTT CTC GCC ATG AAC TTA GGC CTC CCT GAC CTC GTG GCA 4338
Asp Trp Cys Leu Leu Ala Met Asn Leu Gly Leu Pro Asp Leu Val Ala
1425 1430 1435

AAG TAC AAC ACC AAT AAC GGG GCT CCC AAG GAT TTC CTC CCC AGC CCC 4386
Lys Tyr Asn Thr Asn Asn Gly Ala Pro Lys Asp Phe Leu Pro Ser Pro
1440 1445 1450

CTC CAC GCC CTG CTG CGG GAA TGG ACC ACC TAC CCT GAG AGC ACA GTG 4434
Leu His Ala Leu Leu Arg Glu Trp Thr Thr Tyr Pro Glu Ser Thr Val
1455 1460 1465

GGC ACC CTC ATG TCC AAA CTG AGG GAG CTG GGT CGC CGG GAT GCC GCA 4482
Gly Thr Leu Met Ser Lys Leu Arg Glu Leu Gly Arg Arg Asp Ala Ala
1470 1475 1480 1485

GAC CTT TTG CTG AAG GCA TCC TCT GTG TTC AAA ATC AAC CTG GAT GGC 4530
Asp Leu Leu Leu Lys Ala Ser Ser Val Phe Lys Ile Asn Leu Asp Gly
1490 1495 1500

AAT GGC CAG GAG GCC TAT GCC TCG AGC TGC AAC AGC GGC ACC TCT TAC 4578
Asn Gly Gln Glu Ala Tyr Ala Ser Ser Cys Asn Ser Gly Thr Ser Tyr
1505 1510 1515

AAT TCC ATT AGC TCT GTT GTA TCC CGG TGAGGGCAGC CTCTGGCTTG 4625
Asn Ser Ile Ser Ser Val Val Ser Arg
1520 1525

GACAGGGTCT GTTTGGACTG CAGAACCAAG GGGGTGATGT AGCCCATCCT TCCCTTTGGA 4685

GATGCTGAGG GTGTTTCTTC CTGCACCCAC AGCCAGGGGG ATGCCACTCC TCCCTCCGGC 4745

TTGACCTGTT TCTCTGCCGC TACCTCCCTC CCCGTCTCAT TCCGTTGTCT GTGGATGGTC 4805

ATTGCAGTTT AAGAGCAGAA CAGATCTTTT ACTTTGGCCG CTTGAAAAGC TAGTGTACCT 4865

CCTCTCAGTG TTTTGGACTC CATCTCTCAT CCTCCAGTAC CTTGCTTCTT ACTGATAATT 4925

TTGCTGGAAT TCCTAACTTT TCAATGACAT TTTTTTTAAC TATCATATTG ATTGTCCTTT 4985

AAAAAAGAAA AGTGCATATT TATCCAAAAT GTGTATTTCT TATACGCTTT TCTGTGTTAT 5045

ACCATTTCCT CAGCTTATCT CTTTTATATT TGTAGGAGAA ACTCCCATGT ATGGAATCCC 5105

ACTGTATGAT TTATAAACAG ACAATATGTG AGTGCCTTTT GCAGAAGAGG GTGTGTTTGA 5165

AATCATCGGA GTCAGCCAGG AGCTGTCACC AAGGAAACGC TACCTCTCTG TCCCTTGCTG 5225

TATGCTGATC ATCGCCAGAG GTGCTTCACC CTGAGTTTTG TTTTGTATTG TTTTCTGACA 5285

GTTTTTCTGT TTTGTTTGGC AAGGAAAGGG GAGAAGGGAA TCCTCCTCCA GGGTGATTTT 5345

ATGATCAGTG TTGTTGCTCT AGGAAGACAT TTTTCCGTTT GCTTTTGTTC CAATGTCAAT 5405

GTGAACGTCC ACATGAAACC TACACACTGT CATGCTTCAT CATTCCCTCT CATCTCAGGT 5465

AGAAGGTTGA CACAGTTGTA GGGTTACAGA GACCTATGTA AGAATTCAGA AGACCCCTGA 5525

CTCATCATTT GTGGCAGTCC CTTATAATTG GTGCATAGCA GATGGTTTCC ACATTTAGAT 5585

CCTGGTTTCA TAACTTCTCG TACTTGAAGT CTAAAAGCAG AAAATAAAGG AAGCAAGTTT 5645

TCTTCCATGA TTTTAAATTG TGATCGAGTT TTAAATTGAT AGGAGGGAAC ATGTCCTAAT 5705

TCTTCTGTCC TGAGAAGCAT GTAATGTTAA TGTTATATCA TATGTATATA TATATATGCA 5765

CTATGTATAT ACATATATAT TAATACTGGT ATTTTTACTT AATCTATAAA ATGTCGTTAA 5825

AAAGTTGTTT GTTTTTTTCT TTTTTTATAA ATAAACTGTT GCTCGTTAAA AAAAAAAAAA 5885

A 5886

(2) INFORMATION FOR SEQ ID NO: 12:

(i) SEQUENCE CHARACTERISTICS:
(A) LENGTH: 1423 amino acids
(B) TYPE: amino acid
(D) TOPOLOGY: linear

(ii) MOLECULE TYPE: protein
(xi) SEQUENCE DESCRIPTION: SEQ ID NO: 12:

Met Thr Val Phe Arg Gln Glu Asn Val Asp Asp Tyr Tyr Asp Thr Gly
1               5                   10                  15

Glu Glu Leu Gly Ser Gly Gln Phe Ala Val Val Lys Lys Cys Arg Glu
            20                  25                  30

Lys Ser Thr Gly Leu Gln Tyr Pro Ala Lys Phe Ile Lys Lys Arg Arg
        35                  40                  45

Thr Lys Ser Ser Arg Arg Gly Val Ser Arg Glu Asp Ile Glu Arg Glu
    50                  55                  60

Val Ser Ile Leu Lys Glu Ile Gln His Pro Asn Val Ile Thr Leu His
65                  70                  75                  80

Glu Val Tyr Glu Asn Lys Thr Asp Val Ile Leu Ile Leu Glu Leu Val
                85                  90                  95

Ala Gly Gly Glu Leu Phe Asp Phe Leu Ala Glu Lys Glu Ser Leu Thr
            100                 105                 110

Glu Glu Glu Ala Thr Glu Phe Leu Lys Gln Ile Leu Asn Gly Val Tyr
        115                 120                 125

Tyr Leu His Ser Leu Gln Ile Ala His Phe Asp Leu Lys Pro Glu Asn
    130                 135                 140

Ile Met Leu Leu Asp Arg Asn Val Pro Lys Pro Arg Ile Lys Ile Ile
145                 150                 155                 160

Asp Phe Gly Asn Glu Phe Lys Asn Ile Phe Gly Thr Pro Glu Phe Val
                165                 170                 175

Ala Pro Glu Ile Val Asn Tyr Glu Pro Leu Gly Leu Glu Ala Asp Met
            180                 185                 190

Trp Ser Ile Gly Val Ile Thr Tyr Ile Leu Leu Ser Gly Ala Ser Pro
        195                 200                 205

Phe Leu Gly Asp Thr Lys Gln Glu Thr Leu Ala Asn Val Ser Ala Val
    210                 215                 220

Asn Tyr Glu Phe Glu Asp Glu Tyr Phe Ser Asn Thr Ser Ala Leu Ala
225                 230                 235                 240

Lys Asp Phe Ile Arg Arg Leu Leu Val Lys Asp Pro Lys Lys Arg Met
                245                 250                 255

Thr Ile Gln Asp Ser Leu Gln His Pro Trp Ile Lys Pro Lys Asp Thr
            260                 265                 270

Gln Gln Ala Leu Ser Arg Lys Ala Ser Ala Val Asn Met Glu Lys Phe
        275                 280                 285

Lys Lys Phe Ala Ala Arg Lys Lys Trp Lys Gln Ser Val Arg Leu Ile
    290                 295                 300

Ser Leu Cys Gln Arg Leu Ser Arg Ser Phe Leu Ser Arg Ser Asn Met
305                 310                 315                 320

Ser Val Ala Arg Ser Asp Asp Thr Leu Asp Glu Glu Asp Ser Phe Val
                325                 330                 335

Met Lys Ala Ile Ile His Ala Ile Asn Asp Asp Asn Val Pro Gly Leu
            340                 345                 350

Gln His Leu Leu Gly Ser Leu Ser Asn Tyr Asp Val Asn Gln Pro Asn
        355                 360                 365

Lys His Gly Thr Pro Pro Leu Leu Ile Ala Ala Gly Cys Gly Asn Ile
    370                 375                 380

Gln Ile Leu Gln Leu Leu Ile Lys Arg Gly Ser Arg Ile Asp Val Gln
385                 390                 395                 400

Asp Lys Gly Gly Ser Asn Ala Val Tyr Trp Ala Ala Arg His Gly His
                405                 410                 415

Val Asp Thr Leu Lys Phe Leu Ser Glu Asn Lys Cys Pro Leu Asp Val
            420                 425                 430

Lys Asp Lys Ser Gly Glu Met Ala Leu His Val Ala Ala Arg Tyr Gly
        435                 440                 445

His Ala Asp Val Ala Gln Val Thr Cys Ala Ala Ser Ala Gln Ile Pro
    450                 455                 460

Ile Ser Arg Thr Lys Glu Glu Glu Thr Pro Leu His Cys Ala Ala Trp
465                 470                 475                 480

His Gly Tyr Tyr Ser Val Ala Lys Ala Leu Cys Glu Ala Gly Cys Asn
                485                 490                 495

Val Asn Ile Lys Asn Arg Glu Gly Glu Thr Pro Leu Leu Thr Ala Ser
            500                 505                 510

Ala Arg Gly Tyr His Asp Ile Val Glu Cys Leu Ala Glu His Gly Ala
        515                 520                 525

Asp Leu Asn Ala Cys Asp Lys Asp Gly His Ile Ala Leu His Leu Ala
    530                 535                 540

Val Arg Arg Cys Gln Met Glu Val Ile Lys Thr Leu Leu Ser Gln Gly
545                 550                 555                 560

Cys Phe Val Asp Tyr Gln Asp Arg His Gly Asn Thr Pro Leu His Val
                565                 570                 575

Ala Cys Lys Asp Gly Asn Met Pro Ile Val Val Ala Leu Cys Glu Ala
            580                 585                 590

Asn Cys Asn Leu Asp Ile Ser Asn Lys Tyr Gly Arg Thr Pro Leu His
        595                 600                 605

Leu Ala Ala Asn Asn Gly Ile Leu Asp Val Val Arg Tyr Leu Cys Leu
    610                 615                 620

Met Gly Ala Ser Val Glu Ala Leu Thr Thr Asp Gly Lys Thr Ala Glu
625                 630                 635                 640

Asp Leu Ala Arg Ser Glu Gln His Glu His Val Ala Gly Leu Leu Ala
                645                 650                 655

Arg Leu Arg Lys Asp Thr His Arg Gly Leu Phe Ile Gln Gln Leu Arg
            660                 665                 670

Pro Thr Gln Asn Leu Gln Pro Arg Ile Lys Leu Lys Leu Phe Gly His
        675                 680                 685

Ser Gly Ser Gly Lys Thr Thr Leu Val Glu Ser Leu Lys Cys Gly Leu
    690                 695                 700

Leu Arg Ser Phe Phe Arg Arg Arg Arg Pro Arg Leu Ser Ser Thr Asn
705                 710                 715                 720

Ser Ser Arg Phe Pro Pro Ser Pro Leu Ala Ser Lys Pro Thr Val Ser
                725                 730                 735

Val Ser Ile Asn Asn Leu Tyr Pro Gly Cys Glu Asn Val Ser Val Arg
            740                 745                 750

Ser Arg Ser Met Met Phe Glu Pro Gly Leu Thr Lys Gly Met Leu Glu
        755                 760                 765

Val Phe Val Ala Pro Thr His His Pro His Cys Ser Ala Asp Asp Gln
    770                 775                 780

Ser Thr Lys Ala Ile Asp Ile Gln Asn Ala Tyr Leu Asn Gly Val Gly
785                 790                 795                 800

Asp Phe Ser Val Trp Glu Phe Ser Gly Asn Pro Val Tyr Phe Cys Cys
                805                 810                 815

Tyr Asp Tyr Phe Ala Ala Asn Asp Pro Thr Ser Ile His Val Val Val
            820                 825                 830

Phe Ser Leu Glu Glu Pro Tyr Glu Ile Gln Leu Asn Pro Val Ile Phe
        835                 840                 845

Trp Leu Ser Phe Leu Lys Ser Leu Val Pro Val Glu Glu Pro Ile Ala
    850                 855                 860

Phe Gly Gly Lys Leu Lys Asn Pro Leu Gln Val Val Leu Val Ala Thr
865                 870                 875                 880

His Ala Asp Ile Met Asn Val Pro Arg Pro Ala Gly Gly Glu Phe Gly
                885                 890                 895

Tyr Asp Lys Asp Thr Ser Leu Leu Lys Glu Ile Arg Asn Arg Phe Gly
            900                 905                 910

Asn Asp Leu His Ile Ser Asn Lys Leu Phe Val Leu Asp Ala Gly Ala
        915                 920                 925

Ser Gly Ser Lys Asp Met Lys Val Leu Arg Asn His Leu Gln Glu Ile
    930                 935                 940

Arg Ser Gln Ile Val Ser Val Cys Pro Pro Met Thr His Leu Cys Glu
945                 950                 955                 960

Lys Ile Ile Ser Thr Leu Pro Ser Trp Arg Lys Leu Asn Gly Pro Asn
                965                 970                 975

Gln Leu Met Ser Leu Gln Gln Phe Val Tyr Asp Val Gln Asp Gln Leu
            980                 985                 990

Asn Pro Leu Ala Ser Glu Glu Asp Leu Arg Arg Ile Ala Gln Gln Leu
        995                 1000                1005

His Ser Thr Gly Glu Ile Asn Ile Met Gln Ser Glu Thr Val Gln Asp
    1010                1015                1020

Val Leu Leu Leu Asp Pro Arg Trp Leu Cys Thr Asn Val Leu Gly Lys
1025                1030                1035                1040

Leu Leu Ser Val Glu Thr Pro Arg Ala Leu His His Tyr Arg Gly Arg
                1045                1050                1055

Tyr Thr Val Glu Asp Ile Gln Arg Leu Val Pro Asp Ser Asp Val Glu
            1060                1065                1070

Glu Leu Leu Gln Ile Leu Asp Ala Met Asp Ile Cys Ala Arg Asp Leu
        1075                1080                1085

Ser Ser Gly Thr Met Val Asp Val Pro Ala Leu Ile Lys Thr Asp Asn
    1090                1095                1100

Leu His Arg Ser Trp Ala Asp Glu Glu Asp Glu Val Met Val Tyr Gly
1105                1110                1115                1120

Gly Val Arg Ile Val Pro Val Glu His Leu Thr Pro Phe Pro Cys Gly
                1125                1130                1135

Ile Phe His Lys Val Gln Val Asn Leu Cys Arg Trp Ile His Gln Gln
            1140                1145                1150

Ser Thr Glu Gly Asp Ala Asp Ile Arg Leu Trp Val Asn Gly Cys Lys
        1155                1160                1165

Leu Ala Asn Arg Gly Ala Glu Leu Leu Val Leu Leu Val Asn His Gly
    1170                1175                1180

Gln Gly Ile Glu Val Gln Val Arg Gly Leu Glu Thr Glu Lys Ile Lys
1185                1190                1195                1200

Cys Cys Leu Leu Leu Asp Ser Val Cys Ser Thr Ile Glu Asn Val Met
                1205                1210                1215

Ala Thr Thr Leu Pro Gly Leu Leu Thr Val Lys His Tyr Leu Ser Pro
            1220                1225                1230

Gln Gln Leu Arg Glu His His Glu Pro Val Met Ile Tyr Gln Pro Arg
        1235                1240                1245

Asp Phe Phe Arg Ala Gln Thr Leu Lys Glu Thr Ser Leu Thr Asn Thr
    1250                1255                1260

Met Gly Gly Tyr Lys Glu Ser Phe Ser Ser Ile Met Cys Phe Gly Cys
1265                1270                1275                1280

His Asp Val Tyr Ser Gln Ala Ser Leu Gly Met Asp Ile His Ala Ser
                1285                1290                1295

Asp Leu Asn Leu Leu Thr Arg Arg Lys Leu Ser Arg Leu Leu Asp Pro
            1300                1305                1310

Pro Asp Pro Leu Gly Lys Asp Trp Cys Leu Leu Ala Met Asn Leu Gly
        1315                1320                1325

Leu Pro Asp Leu Val Ala Lys Tyr Asn Thr Asn Asn Gly Ala Pro Lys
    1330                1335                1340

Asp Phe Leu Pro Ser Pro Leu His Ala Leu Leu Arg Glu Trp Thr Thr
1345                1350                1355                1360

Tyr Pro Glu Ser Thr Val Gly Thr Leu Met Ser Lys Leu Arg Glu Leu
                1365                1370                1375

Gly Arg Arg Asp Ala Ala Asp Leu Leu Leu Lys Ala Ser Ser Val Phe
            1380                1385                1390

Lys Ile Asn Leu Asp Gly Asn Gly Gln Glu Ala Tyr Ala Ser Ser Cys
        1395                1400                1405

Asn Ser Gly Thr Ser Tyr Asn Ser Ile Ser Ser Val Val Ser Arg
    1410                1415                1420

(2) INFORMATION FOR SEQ ID NO: 13:

     (i) SEQUENCE CHARACTERISTICS:
          (A) LENGTH: 1568 base pairs
          (B) TYPE: nucleic acid
          (C) STRANDEDNESS: double
          (D) TOPOLOGY: linear

    (ii) MOLECULE TYPE: DNA (genomic)

    (ix) FEATURE:
          (A) NAME/KEY: CDS
          (B) LOCATION:74..1270
          (D) OTHER INFORMATION:/note= "prevention of IFN-2
                promoted cell death"

    (xi) SEQUENCE DESCRIPTION: SEQ ID NO: 13:

GAATTCCGCC GGCCCCAGGC AGCGTGTGTC GGTCGCCTAG GCTGGAGAAC TAGTCCTCGA      60

CTCACGTGCA AGG ATG ATG CTG AAA GGA ATA ACA AGG CTT ATC TCT AGG        109
               Met Met Leu Lys Gly Ile Thr Arg Leu Ile Ser Arg
                   1425                1430                1435

ATC CAT AAG TTG GAC CCT GGG CGT TTT TTA CAC ATG GGG ACC CAG GCT       157
Ile His Lys Leu Asp Pro Gly Arg Phe Leu His Met Gly Thr Gln Ala
                1440                1445                1450

CGC CAA AGC ATT GCT GCT CAC CTA GAT AAC CAG GTT CCA GTT GAG AGT       205
Arg Gln Ser Ile Ala Ala His Leu Asp Asn Gln Val Pro Val Glu Ser
            1455                1460                1465

CCG AGA GCT ATT TCC CGC ACC AAT GAG AAT GAC CCG GCC AAG CAT GGG       253
Pro Arg Ala Ile Ser Arg Thr Asn Glu Asn Asp Pro Ala Lys His Gly
        1470                1475                1480

GAT CAG CAC GAG GGT CAG CAC TAC AAC ATC TCC CCC CAG GAT TTG GAG       301
Asp Gln His Glu Gly Gln His Tyr Asn Ile Ser Pro Gln Asp Leu Glu
    1485                1490                1495

ACT GTA TTT CCC CAT GGC CTT CCT CCT CGC TTT GTG ATG CAG GTG AAG       349
Thr Val Phe Pro His Gly Leu Pro Pro Arg Phe Val Met Gln Val Lys
1500                1505                1510                1515

ACA TTC AGT GAA GCT TGC CTG ATG GTA AGG AAA CCA GCC CTA GAA CTT       397
Thr Phe Ser Glu Ala Cys Leu Met Val Arg Lys Pro Ala Leu Glu Leu
                1520                1525                1530

CTG CAT TAC CTG AAA AAC ACC AGT TTT GCT TAT CCA GCT ATA CGA TAT 445
Leu His Tyr Leu Lys Asn Thr Ser Phe Ala Tyr Pro Ala Ile Arg Tyr
1535 1540 1545

CTT CTG TAT GGA GAG AAG GGA ACA GGA AAA ACC CTA AGT CTT TGC CAT 493
Leu Leu Tyr Gly Glu Lys Gly Thr Gly Lys Thr Leu Ser Leu Cys His
1550 1555 1560

GTT ATT CAT TTC TGT GCA AAA CAG GAC TGG CTG ATA CTA CAT ATT CCA 541
Val Ile His Phe Cys Ala Lys Gln Asp Trp Leu Ile Leu His Ile Pro
1565 1570 1575

GAT GCT CAT CTT TGG GTG AAA AAT TGT CGG GAT CTT CTG CAG TCC AGC 589
Asp Ala His Leu Trp Val Lys Asn Cys Arg Asp Leu Leu Gln Ser Ser
1580 1585 1590 1595

TAC AAC AAA CAG CGC TTT GAT CAA CCT TTA GAG GCT TCA ACC TGG CTG 637
Tyr Asn Lys Gln Arg Phe Asp Gln Pro Leu Glu Ala Ser Thr Trp Leu
1600 1605 1610

AAG AAT TTC AAA ACT ACA AAT GAG CGC TTC CTG AAC CAG ATA AAA GTT 685
Lys Asn Phe Lys Thr Thr Asn Glu Arg Phe Leu Asn Gln Ile Lys Val
1615 1620 1625

CAA GAG AAG TAT GTC TGG AAT AAG AGA GAA AGC ACT GAG AAA GGG AGT 733
Gln Glu Lys Tyr Val Trp Asn Lys Arg Glu Ser Thr Glu Lys Gly Ser
1630 1635 1640

CCT CTG GGA GAA GTG GTT GAA CAG GGC ATA ACA CGG GTG AGG AAC GCC 781
Pro Leu Gly Glu Val Val Glu Gln Gly Ile Thr Arg Val Arg Asn Ala
1645 1650 1655

ACA GAT GCA GTT GGA ATT GTG CTG AAA GAG CTA AAG AGG CAA AGT TCT 829
Thr Asp Ala Val Gly Ile Val Leu Lys Glu Leu Lys Arg Gln Ser Ser
1660 1665 1670 1675

TTG GGT ATG TTT CAC CTC CTA GTG GCC GTG GAT GGA ATC AAT GCT CTT 877
Leu Gly Met Phe His Leu Leu Val Ala Val Asp Gly Ile Asn Ala Leu
1680 1685 1690

TGG GGA AGA ACC ACT CTG AAA AGA GAA GAT AAA AGC CCG ATT GCC CCC 925
Trp Gly Arg Thr Thr Leu Lys Arg Glu Asp Lys Ser Pro Ile Ala Pro
1695 1700 1705

GAG GAA TTA GCA CTT GTT CAC AAC TTG AGG AAA ATG ATG AAA AAT GAT 973
Glu Glu Leu Ala Leu Val His Asn Leu Arg Lys Met Met Lys Asn Asp
1710 1715 1720

TGG CAT GGA GGC GCC ATT GTG TCG GCT TTG AGC CAG ACT GGG TCT CTC 1021
Trp His Gly Gly Ala Ile Val Ser Ala Leu Ser Gln Thr Gly Ser Leu
1725 1730 1735

TTT AAG CCC CGG AAA GCC TAT CTG CCC CAG GAG TTG CTG GGA AAG GAA 1069
Phe Lys Pro Arg Lys Ala Tyr Leu Pro Gln Glu Leu Leu Gly Lys Glu
1740 1745 1750 1755

GGA TTT GAT GCC CTG GAT CCC TTT ATT CCC ATC CTG GTT TCC AAC TAT 1117
Gly Phe Asp Ala Leu Asp Pro Phe Ile Pro Ile Leu Val Ser Asn Tyr
1760 1765 1770

AAC CCA AAG GAA TTT GAA AGT TGT ATT CAG TAT TAT TTG GAA AAC AAT      1165
Asn Pro Lys Glu Phe Glu Ser Cys Ile Gln Tyr Tyr Leu Glu Asn Asn
        1775                1780                1785

TGG CTT CAA CAT GAG AAA GCT CCT ACA GAA GAA GGG AAA AAA GAG CTG      1213
Trp Leu Gln His Glu Lys Ala Pro Thr Glu Glu Gly Lys Lys Glu Leu
    1790                1795                1800

CTG TTC CTA AGT AAC GCG AAC CCC TCG CTG CTG GAG CGG CAC TGT GCC      1261
Leu Phe Leu Ser Asn Ala Asn Pro Ser Leu Leu Glu Arg His Cys Ala
1805                1810                1815

TAC CTC TAA GCCAAGATCA CAGCATGTGA GGAAGACAGT GGACATCTGC              1310
Tyr Leu  *
1820

TTTATGCTGG ACCCAGTAAG ATGAGGAAGT CGGGCAGTAC ACAGGAAGAG GAGCCAGGCC    1370

CTTGTACCTA TGGGATTGGA CAGGACTGCA GTTGGCTCTG GACCTGCATT AAAATGGGTT    1430

TCACTGTGAA TGCGTGACAA TAAGATATTC CCTTGTTCCT AAAACTTTAT ATCAGTTTAT    1490

TGGATGTGGG TTTTTCACAT TTAAGATAAT TATGGCTCTT TTCCTAAAAA ATAAAATATC    1550

TTTCTAAAAA AAAAAAAA                                                  1568

(2) INFORMATION FOR SEQ ID NO: 14:

      (i) SEQUENCE CHARACTERISTICS:
         (A) LENGTH: 399 amino acids
         (B) TYPE: amino acid
         (D) TOPOLOGY: linear

    (ii) MOLECULE TYPE: protein
    (xi) SEQUENCE DESCRIPTION: SEQ ID NO: 14:

Met Met Leu Lys Gly Ile Thr Arg Leu Ile Ser Arg Ile His Lys Leu
  1               5                  10                  15

Asp Pro Gly Arg Phe Leu His Met Gly Thr Gln Ala Arg Gln Ser Ile
             20                  25                  30

Ala Ala His Leu Asp Asn Gln Val Pro Val Glu Ser Pro Arg Ala Ile
         35                  40                  45

Ser Arg Thr Asn Glu Asn Asp Pro Ala Lys His Gly Asp Gln His Glu
     50                  55                  60

Gly Gln His Tyr Asn Ile Ser Pro Gln Asp Leu Glu Thr Val Phe Pro
 65                  70                  75                  80

His Gly Leu Pro Pro Arg Phe Val Met Gln Val Lys Thr Phe Ser Glu
                 85                  90                  95

Ala Cys Leu Met Val Arg Lys Pro Ala Leu Glu Leu Leu His Tyr Leu
            100                 105                 110

Lys Asn Thr Ser Phe Ala Tyr Pro Ala Ile Arg Tyr Leu Leu Tyr Gly
        115                 120                 125

Glu Lys Gly Thr Gly Lys Thr Leu Ser Leu Cys His Val Ile His Phe

130                 135                 140

Cys Ala Lys Gln Asp Trp Leu Ile Leu His Ile Pro Asp Ala His Leu
145                 150                 155                 160

Trp Val Lys Asn Cys Arg Asp Leu Leu Gln Ser Ser Tyr Asn Lys Gln
                165                 170                 175

Arg Phe Asp Gln Pro Leu Glu Ala Ser Thr Trp Leu Lys Asn Phe Lys
            180                 185                 190

Thr Thr Asn Glu Arg Phe Leu Asn Gln Ile Lys Val Gln Glu Lys Tyr
        195                 200                 205

Val Trp Asn Lys Arg Glu Ser Thr Glu Lys Gly Ser Pro Leu Gly Glu
    210                 215                 220

Val Val Glu Gln Gly Ile Thr Arg Val Arg Asn Ala Thr Asp Ala Val
225                 230                 235                 240

Gly Ile Val Leu Lys Glu Leu Lys Arg Gln Ser Ser Leu Gly Met Phe
                245                 250                 255

His Leu Leu Val Ala Val Asp Gly Ile Asn Ala Leu Trp Gly Arg Thr
            260                 265                 270

Thr Leu Lys Arg Glu Asp Lys Ser Pro Ile Ala Pro Glu Glu Leu Ala
        275                 280                 285

Leu Val His Asn Leu Arg Lys Met Met Lys Asn Asp Trp His Gly Gly
    290                 295                 300

Ala Ile Val Ser Ala Leu Ser Gln Thr Gly Ser Leu Phe Lys Pro Arg
305                 310                 315                 320

Lys Ala Tyr Leu Pro Gln Glu Leu Leu Gly Lys Glu Gly Phe Asp Ala
                325                 330                 335

Leu Asp Pro Phe Ile Pro Ile Leu Val Ser Asn Tyr Asn Pro Lys Glu
            340                 345                 350

Phe Glu Ser Cys Ile Gln Tyr Tyr Leu Glu Asn Asn Trp Leu Gln His
        355                 360                 365

Glu Lys Ala Pro Thr Glu Glu Gly Lys Lys Glu Leu Leu Phe Leu Ser
    370                 375                 380

Asn Ala Asn Pro Ser Leu Leu Glu Arg His Cys Ala Tyr Leu  *
385                 390                 395

(2) INFORMATION FOR SEQ ID NO: 15:

     (i) SEQUENCE CHARACTERISTICS:
          (A) LENGTH: 132 base pairs
          (B) TYPE: nucleic acid
          (C) STRANDEDNESS: double
          (D) TOPOLOGY: linear

    (ii) MOLECULE TYPE: DNA (genomic)

(xi) SEQUENCE DESCRIPTION: SEQ ID NO: 15:

CTAGATGAGG CAGATATAAG AGTCATGGAA AAAAGGACAG AGAAAAAAAA CAGACAAATC 60

AGTTGTCAGT ATCCATGGCC TCTGATTCTG TCTCAACCAT GAAACAGAAG TGACACATAT 120

ACCTGCTAAA AG 132

(2) INFORMATION FOR SEQ ID NO: 16:

(i) SEQUENCE CHARACTERISTICS:
(A) LENGTH: 255 base pairs
(B) TYPE: nucleic acid
(C) STRANDEDNESS: double
(D) TOPOLOGY: linear

(ii) MOLECULE TYPE: DNA (genomic)

(xi) SEQUENCE DESCRIPTION: SEQ ID NO: 16:

AACTCAGTCC CTTTCCTTCC AAAATCTCCA ACATGCGGTC CTTATTCTGA TCAATTTCTG 60

GGAGCATTTT CTGCATATTG ACCTTGCTTT GTTGAAAAAG TTCTGTTAAC CATTCTCGAT 120

CTTGTAATTT AGCTAACTGC TGAAGACAAA GTAGGAAGAG AGGAAAATGG GTGCCACTTT 180

CTAGTGGTTG AGCTAGTTCT GAAATGCTCA CCAGCTCTGA AATGATGGCA CGAGCTGCAA 240

ACTGTGCTAA ATAGC 255

(2) INFORMATION FOR SEQ ID NO: 17:

(i) SEQUENCE CHARACTERISTICS:
(A) LENGTH: 2038 base pairs
(B) TYPE: nucleic acid
(C) STRANDEDNESS: double
(D) TOPOLOGY: linear

(ii) MOLECULE TYPE: DNA (genomic)

(ix) FEATURE:
(A) NAME/KEY: CDS
(B) LOCATION:52..1230

(xi) SEQUENCE DESCRIPTION: SEQ ID NO: 17:

GGCTATAAGC GCACGGCCTC GGCGACCCTC TCCGACCCGG CCGCCGCCGC C ATG CAG 57
Met Gln
400

CCC TCC AGC CTT CTG CCG CTC GCC CTC TGC CTG CTG GCT GCA CCC GCC 105
Pro Ser Ser Leu Leu Pro Leu Ala Leu Cys Leu Leu Ala Ala Pro Ala
405 410 415

TCC GCG CTC GTC AGG ATC CCG CTG CAC AAG TTC ACG TCC ATC CGC CGG 153
Ser Ala Leu Val Arg Ile Pro Leu His Lys Phe Thr Ser Ile Arg Arg
420 425 430

ACC ATG TCG GAG GTT GGG GGC TCT GTG GAG GAC CTG ATT GCC AAA GGC 201
Thr Met Ser Glu Val Gly Gly Ser Val Glu Asp Leu Ile Ala Lys Gly
435 440 445

CCC GTC TCA AAG TAC TCC CAG GCG GTG CCA GCC GTG ACC GAG GGG CCC 249
Pro Val Ser Lys Tyr Ser Gln Ala Val Pro Ala Val Thr Glu Gly Pro
450 455 460 465

ATT CCC GAG GTG CTC AAG AAC TAC ATG GAC GCC CAG TAC TAC GGG GAG 297
Ile Pro Glu Val Leu Lys Asn Tyr Met Asp Ala Gln Tyr Tyr Gly Glu
470 475 480

ATT GGC ATC GGG ACG CCC CCC CAG TGC TTC ACA GTC GTC TTC GAC ACG 345
Ile Gly Ile Gly Thr Pro Pro Gln Cys Phe Thr Val Val Phe Asp Thr
485 490 495

GGC TCC TCC AAC CTG TGG GTC CCC TCC ATC CAC TGC AAA CTG CTG GAC 393
Gly Ser Ser Asn Leu Trp Val Pro Ser Ile His Cys Lys Leu Leu Asp
500 505 510

ATC GCT TGC TGG ATC CAC CAC AAG TAC AAC AGC GAC AAG TCC AGC ACC 441
Ile Ala Cys Trp Ile His His Lys Tyr Asn Ser Asp Lys Ser Ser Thr
515 520 525

TAC GTG AAG AAT GGT ACC TCG TTT GAC ATC CAC TAT GGC TCG GGC AGC 489
Tyr Val Lys Asn Gly Thr Ser Phe Asp Ile His Tyr Gly Ser Gly Ser
530 535 540 545

CTC TCC GGG TAC CTG AGC CAG GAC ACT GTG TCG GTG CCC TGC CAG TCA 537
Leu Ser Gly Tyr Leu Ser Gln Asp Thr Val Ser Val Pro Cys Gln Ser
550 555 560

GCG TCG TCA GCC TCT GCC CTG GGC GGT GTC AAA GTG GAG AGG CAG GTC 585
Ala Ser Ser Ala Ser Ala Leu Gly Gly Val Lys Val Glu Arg Gln Val
565 570 575

TTT GGG GAG GCC ACC AAG CAG CCA GGC ATC ACC TTC ATC GCA GCC AAG 633
Phe Gly Glu Ala Thr Lys Gln Pro Gly Ile Thr Phe Ile Ala Ala Lys
580 585 590

TTC GAT GGC ATC CTG GGC ATG GCC TAC CCC CGC ATC TCC GTC AAC AAC 681
Phe Asp Gly Ile Leu Gly Met Ala Tyr Pro Arg Ile Ser Val Asn Asn
595 600 605

GTG CTG CCC GTC TTC GAC AAC CTG ATG CAG CAG AAG CTG GTG GAC CAG 729
Val Leu Pro Val Phe Asp Asn Leu Met Gln Gln Lys Leu Val Asp Gln
610 615 620 625

AAC ATC TTC TCC TTC TAC CTG AGC AGG GAC CCA GAT GCG CAG CCT GGG 777
Asn Ile Phe Ser Phe Tyr Leu Ser Arg Asp Pro Asp Ala Gln Pro Gly
630 635 640

GGT GAG CTG ATG CTG GGT GGC ACA GAC TCC AAG TAT TAC AAG GGT TCT 825
Gly Glu Leu Met Leu Gly Gly Thr Asp Ser Lys Tyr Tyr Lys Gly Ser
645 650 655

CTG TCC TAC CTG AAT GTC ACC CGC AAG GCC TAC TGG CAG GTC CAC CTG 873
Leu Ser Tyr Leu Asn Val Thr Arg Lys Ala Tyr Trp Gln Val His Leu
660 665 670

GAC CAG GTG GAG GTG GCC AGC GGG CTG ACC CTG TGC AAG GAG GGC TGT 921
Asp Gln Val Glu Val Ala Ser Gly Leu Thr Leu Cys Lys Glu Gly Cys
675 680 685

GAG GCC ATT GTG GAC ACA GGC ACT TCC CTC ATG GTG GGC CCG GTG GAT 969
Glu Ala Ile Val Asp Thr Gly Thr Ser Leu Met Val Gly Pro Val Asp
690 695 700 705

GAG GTG CGC GAG CTG CAG AAG GCC ATC GGG GCC GTG CCG CTG ATT CAG 1017
Glu Val Arg Glu Leu Gln Lys Ala Ile Gly Ala Val Pro Leu Ile Gln
710 715 720

GGC GAG TAC ATG ATC CCC TGT GAG AAG GTG TCC ACC CTG CCC GCG ATC 1065
Gly Glu Tyr Met Ile Pro Cys Glu Lys Val Ser Thr Leu Pro Ala Ile
725 730 735

ACA CTG AAG CTG GGA GGC AAA GGC TAC AAG CTG TCC CCA GAG GAC TAC 1113
Thr Leu Lys Leu Gly Gly Lys Gly Tyr Lys Leu Ser Pro Glu Asp Tyr
740 745 750

ACG CTC AAG GTG TCG CAG GCC GGG AAG ACC CTC TGC CTG AGC GGC TTC 1161
Thr Leu Lys Val Ser Gln Ala Gly Lys Thr Leu Cys Leu Ser Gly Phe
755 760 765

ATG GGC ATG GAC ATC CCG CCA CCC AGC GGG CCA CTC TGG ATC CTG GGC 1209
Met Gly Met Asp Ile Pro Pro Pro Ser Gly Pro Leu Trp Ile Leu Gly
770 775 780 785

GAC GTC TTC ATC GGC CGC TAC TACACTGTGT TTGACCGTGA CAACAACAGG 1260
Asp Val Phe Ile Gly Arg Tyr
790

GTGGGCTTCG CCGAGGCTGC CCGCCTCTAG TTCCCAAGGC GTCCGCGCGC CAGCACAGAA 1320

ACAGAGGAGA GTCCCAGAGC AGGAGGCCCC TGGCCCAGCG GCCCCTCCCA CACACACCCA 1380

CACACTCGCC CGCCCACTGT CCTGGGCGCC CTGGAAGCCG GCGGCCCAAG CCCGACTTGC 1440

TGTTTTGTTC TGTGGTTTTC CCCTCCCTGG GTTCAGAAAT GCTGCCTGCC TGTCTGTCTC 1500

TCCATCTGTT TGGTGGGGGT AGAGCTGATC CAGAGCACAG ATCTGTTTCG TGCATTGGAA 1560

GACCCCACCC AAGCTTGGCA GCCGAGCTCG TGTATCCTGG GGCTCCCTTC ATCTCCAGGG 1620

AGTCCCCTCC CCGGCCCTAC CAGCGCCCGC TGGGCTGAGC CCCTACCCCA CACCAGGCCG 1680

TCCTCCCGGG CCCTCCCTTG GAAACCTGCC CTGCCTGAGG GCCCCTCTGC CCAGCTTGGG 1740

CCCAGCTGGG CTCTGCCACC CTACCTGTTC AGTGTCCCGG GCCCGTTGAG GATGAGGCCG 1800

CTAGAGGCCT GAGGATGAGC TGGAAGGAGT GAGAGGGGAC AAAACCCACC TTGTTGGAGC 1860

CTGCAGGGTG GTGCTGGGAC TGAGCCAGTC CCAGGGGCAT GTATTGGCCT GGAGGTGGGG 1920

TTGGGATTGG GGGCTGGTGC CAGCCTTCCT CTGCAGCTGA CCTCTGTTGT CCTCCCCTTG 1980

GGCGGCTGAG AGCCCCAGCT GACATGGAAA TACAGTTGTT GGCCTCCGGC CTCCCCTC 2038

(2) INFORMATION FOR SEQ ID NO: 18:

(i) SEQUENCE CHARACTERISTICS:
(A) LENGTH: 393 amino acids
(B) TYPE: amino acid
(D) TOPOLOGY: linear

(ii) MOLECULE TYPE: protein
(xi) SEQUENCE DESCRIPTION: SEQ ID NO: 18:

Met Gln Pro Ser Ser Leu Leu Pro Leu Ala Leu Cys Leu Leu Ala Ala
1               5                   10                  15

Pro Ala Ser Ala Leu Val Arg Ile Pro Leu His Lys Phe Thr Ser Ile
            20                  25                  30

Arg Arg Thr Met Ser Glu Val Gly Gly Ser Val Glu Asp Leu Ile Ala
        35                  40                  45

Lys Gly Pro Val Ser Lys Tyr Ser Gln Ala Val Pro Ala Val Thr Glu
    50                  55                  60

Gly Pro Ile Pro Glu Val Leu Lys Asn Tyr Met Asp Ala Gln Tyr Tyr
65                  70                  75                  80

Gly Glu Ile Gly Ile Gly Thr Pro Pro Gln Cys Phe Thr Val Val Phe
                85                  90                  95

Asp Thr Gly Ser Ser Asn Leu Trp Val Pro Ser Ile His Cys Lys Leu
            100                 105                 110

Leu Asp Ile Ala Cys Trp Ile His His Lys Tyr Asn Ser Asp Lys Ser
        115                 120                 125

Ser Thr Tyr Val Lys Asn Gly Thr Ser Phe Asp Ile His Tyr Gly Ser
    130                 135                 140

Gly Ser Leu Ser Gly Tyr Leu Ser Gln Asp Thr Val Ser Val Pro Cys
145                 150                 155                 160

Gln Ser Ala Ser Ser Ala Ser Ala Leu Gly Gly Val Lys Val Glu Arg
                165                 170                 175

Gln Val Phe Gly Glu Ala Thr Lys Gln Pro Gly Ile Thr Phe Ile Ala
            180                 185                 190

Ala Lys Phe Asp Gly Ile Leu Gly Met Ala Tyr Pro Arg Ile Ser Val
        195                 200                 205

Asn Asn Val Leu Pro Val Phe Asp Asn Leu Met Gln Gln Lys Leu Val
    210                 215                 220

Asp Gln Asn Ile Phe Ser Phe Tyr Leu Ser Arg Asp Pro Asp Ala Gln
225                 230                 235                 240

Pro Gly Gly Glu Leu Met Leu Gly Gly Thr Asp Ser Lys Tyr Tyr Lys
                245                 250                 255

Gly Ser Leu Ser Tyr Leu Asn Val Thr Arg Lys Ala Tyr Trp Gln Val
            260                 265                 270

His Leu Asp Gln Val Glu Val Ala Ser Gly Leu Thr Leu Cys Lys Glu

275                    280                    285

Gly Cys Glu Ala Ile Val Asp Thr Gly Thr Ser Leu Met Val Gly Pro
    290                    295                    300

Val Asp Glu Val Arg Glu Leu Gln Lys Ala Ile Gly Ala Val Pro Leu
305                    310                    315                    320

Ile Gln Gly Glu Tyr Met Ile Pro Cys Glu Lys Val Ser Thr Leu Pro
                325                    330                    335

Ala Ile Thr Leu Lys Leu Gly Gly Lys Gly Tyr Lys Leu Ser Pro Glu
            340                    345                    350

Asp Tyr Thr Leu Lys Val Ser Gln Ala Gly Lys Thr Leu Cys Leu Ser
        355                    360                    365

Gly Phe Met Gly Met Asp Ile Pro Pro Pro Ser Gly Pro Leu Trp Ile
    370                    375                    380

Leu Gly Asp Val Phe Ile Gly Arg Tyr
385                    390

(2) INFORMATION FOR SEQ ID NO: 19:

    (i) SEQUENCE CHARACTERISTICS:
        (A) LENGTH: 331 amino acids
        (B) TYPE: amino acid
        (C) STRANDEDNESS: double
        (D) TOPOLOGY: linear

    (ii) MOLECULE TYPE: DNA (genomic)

    (xi) SEQUENCE DESCRIPTION: SEQ ID NO: 19:

Met Thr Val Phe Arg Gln Glu Asn Val Asp Asp Tyr Tyr Asp Thr Gly
1               5                   10                  15

Glu Glu Leu Gly Ser Gly Gln Phe Ala Val Val Lys Lys Cys Arg Glu
            20                  25                  30

Lys Ser Thr Gly Leu Gln Tyr Pro Ala Lys Phe Ile Lys Lys Arg Arg
        35                  40                  45

Thr Lys Ser Ser Arg Arg Gly Val Ser Arg Glu Asp Ile Glu Arg Glu
    50                  55                  60

Val Ser Ile Leu Lys Glu Ile Gln His Pro Asn Val Ile Thr Leu His
65                  70                  75                  80

Glu Val Tyr Glu Asn Lys Thr Asp Val Ile Leu Ile Leu Glu Leu Val
                85                  90                  95

Ala Gly Gly Glu Leu Phe Asp Phe Leu Ala Glu Lys Glu Ser Leu Thr
            100                 105                 110

Glu Glu Glu Ala Thr Glu Phe Leu Lys Gln Ile Leu Asn Gly Val Tyr
        115                 120                 125

Tyr Leu His Ser Leu Gln Ile Ala His Phe Asp Leu Lys Pro Glu Asn
    130                 135                 140

Ile Met Leu Leu Asp Arg Asn Val Pro Lys Pro Arg Ile Lys Ile Ile
145                 150                 155                 160

Asp Phe Gly Asn Glu Phe Lys Asn Ile Phe Gly Thr Pro Glu Phe Val
                165                 170                 175

Ala Pro Glu Ile Val Asn Tyr Glu Pro Leu Gly Leu Glu Ala Asp Met
            180                 185                 190

Trp Ser Ile Gly Val Ile Thr Tyr Ile Leu Leu Ser Gly Ala Ser Pro
        195                 200                 205

Phe Leu Gly Asp Thr Lys Gln Glu Thr Leu Ala Asn Val Ser Ala Val
    210                 215                 220

Asn Tyr Glu Phe Glu Asp Glu Tyr Phe Ser Asn Thr Ser Ala Leu Ala
225                 230                 235                 240

Lys Asp Phe Ile Arg Arg Leu Leu Val Lys Asp Pro Lys Lys Arg Met
                245                 250                 255

Thr Ile Gln Asp Ser Leu Gln His Pro Trp Ile Lys Pro Lys Asp Thr
            260                 265                 270

Gln Gln Ala Leu Ser Arg Lys Ala Ser Ala Val Asn Met Glu Lys Phe
        275                 280                 285

Lys Lys Phe Ala Ala Arg Lys Lys Trp Lys Gln Ser Val Arg Leu Ile
    290                 295                 300

Ser Leu Cys Gln Arg Leu Ser Arg Ser Phe Leu Ser Arg Ser Asn Met
305                 310                 315                 320

Ser Val Ala Arg Ser Asp Asp Thr Leu Asp Glu
                325                 330

(2) INFORMATION FOR SEQ ID NO: 20:

    (i) SEQUENCE CHARACTERISTICS:
        (A) LENGTH: 33 amino acids
        (B) TYPE: amino acid
        (C) STRANDEDNESS: double
        (D) TOPOLOGY: linear

    (ii) MOLECULE TYPE: DNA (genomic)

    (ix) FEATURE:
        (A) NAME/KEY: Domain
        (B) LOCATION:1..33
        (D) OTHER INFORMATION:/note= "AR2"

    (xi) SEQUENCE DESCRIPTION: SEQ ID NO: 20:

Asp Val Gln Asp Lys Gly Gly Ser Asn Ala Val Tyr Trp Ala Ala Arg

1               5                   10                  15

His Gly His Val Asp Thr Leu Lys Phe Leu Ser Glu Asn Lys Cys Pro
            20                  25                  30

Leu

(2) INFORMATION FOR SEQ ID NO: 21:

(i) SEQUENCE CHARACTERISTICS:
(A) LENGTH: 33 amino acids
(B) TYPE: amino acid
(C) STRANDEDNESS: double
(D) TOPOLOGY: linear

(ii) MOLECULE TYPE: DNA (genomic)

(ix) FEATURE:
(A) NAME/KEY: Domain
(B) LOCATION:1..33
(D) OTHER INFORMATION:/note= "ar3"

(xi) SEQUENCE DESCRIPTION: SEQ ID NO: 21:

Asp Val Lys Asp Lys Ser Gly Glu Met Ala Leu His Val Ala Ala Arg
1               5                   10                  15

Tyr Gly His Ala Asp Val Ala Gln Val Thr Cys Ala Ala Ser Ala Gln
            20                  25                  30

Ile

(2) INFORMATION FOR SEQ ID NO: 22:

(i) SEQUENCE CHARACTERISTICS:
(A) LENGTH: 33 amino acids
(B) TYPE: amino acid
(C) STRANDEDNESS: double
(D) TOPOLOGY: linear

(ii) MOLECULE TYPE: DNA (genomic)

(ix) FEATURE:
(A) NAME/KEY: Domain
(B) LOCATION:1..33
(D) OTHER INFORMATION:/note= "ar4"

(xi) SEQUENCE DESCRIPTION: SEQ ID NO: 22:

Ile Ser Arg Thr Lys Glu Glu Glu Thr Pro Leu His Cys Ala Ala Trp
1               5                   10                  15

His Gly Tyr Tyr Ser Val Ala Lys Ala Leu Cys Glu Ala Gly Cys Asn
            20                  25                  30

Val

(2) INFORMATION FOR SEQ ID NO: 23:

(i) SEQUENCE CHARACTERISTICS:
(A) LENGTH: 33 amino acids
(B) TYPE: amino acid
(C) STRANDEDNESS: double
(D) TOPOLOGY: linear

(ii) MOLECULE TYPE: DNA (genomic)

(ix) FEATURE:
(A) NAME/KEY: Domain
(B) LOCATION:1..33
(D) OTHER INFORMATION:/note= "ar5"

(xi) SEQUENCE DESCRIPTION: SEQ ID NO: 23:

Asn Ile Lys Asn Arg Glu Gly Glu Thr Pro Leu Leu Thr Ala Ser Ala
1               5                   10                  15

Arg Gly Tyr Met Asp Ile Val Glu Cys Leu Ala Glu His Gly Ala Asp
            20                  25                  30

Leu

(2) INFORMATION FOR SEQ ID NO: 24:

(i) SEQUENCE CHARACTERISTICS:
(A) LENGTH: 33 amino acids
(B) TYPE: amino acid
(C) STRANDEDNESS: double
(D) TOPOLOGY: linear

(ii) MOLECULE TYPE: DNA (genomic)

(ix) FEATURE:
(A) NAME/KEY: Domain
(B) LOCATION:1..33
(D) OTHER INFORMATION:/note= "ar6"

(xi) SEQUENCE DESCRIPTION: SEQ ID NO: 24:

Asn Ala Cys Asp Lys Asp Gly His Ile Ala Leu His Leu Ala Val Arg
1               5                   10                  15

Arg Cys Gln Met Glu Val Ile Lys Thr Leu Leu Ser Gln Gly Cys Phe
            20                  25                  30

Val

(2) INFORMATION FOR SEQ ID NO: 25:

(i) SEQUENCE CHARACTERISTICS:
(A) LENGTH: 33 amino acids
(B) TYPE: amino acid
(C) STRANDEDNESS: double

(D) TOPOLOGY: linear

(ii) MOLECULE TYPE: DNA (genomic)

(ix) FEATURE:
(A) NAME/KEY: Domain
(B) LOCATION:1..33
(D) OTHER INFORMATION:/note= "ar7"

(xi) SEQUENCE DESCRIPTION: SEQ ID NO: 25:

Asp Tyr Gln Asp Arg His Gly Asn Thr Pro Leu His Val Ala Cys Lys
1               5                   10                  15

Asp Gly Asn Met Pro Ile Val Val Ala Leu Cys Glu Ala Asn Cys Asn
            20                  25                  30

Leu

(2) INFORMATION FOR SEQ ID NO: 26:

(i) SEQUENCE CHARACTERISTICS:
(A) LENGTH: 33 amino acids
(B) TYPE: amino acid
(C) STRANDEDNESS: double
(D) TOPOLOGY: linear

(ii) MOLECULE TYPE: DNA (genomic)

(ix) FEATURE:
(A) NAME/KEY: Domain
(B) LOCATION:1..33
(D) OTHER INFORMATION:/note= "ar8"

(xi) SEQUENCE DESCRIPTION: SEQ ID NO: 26:

Asp Ile Ser Asn Lys Tyr Gly Arg Thr Pro Leu His Leu Ala Ala Asn
1               5                   10                  15

Asn Gly Ile Leu Asp Val Val Arg Tyr Leu Cys Leu Met Gly Ala Ser
            20                  25                  30

Val